# EUROPÄISCHE PATENTSCHRIFT

(11) **EP 0 785 932 B1**
(45) Veröffentlichungstag und Bekanntmachung des Hinweises auf die Patenterteilung: **29.12.1999**
(21) Anmeldenummer: 95935894.6
(22) Anmeldetag: 02.10.1995
(51) Int. Cl.: C07D 403/04, A61K 31/495

(54) **PYRROLYL-TETRAHYDROBENZOCHINOXALINDIONE, IHRE HERSTELLUNG UND VERWENDUNG ALS GLUTAMATREZEPTOR-ANTAGONISTEN**
PYRROLYL TETRAHYDROBENZOQUINOXALINE DIONES, THEIR PREPARATION AND USE AS GLUTAMATE RECEPTOR ANTAGONISTS
PYRROLYLTETRAHYDROBENZOQUINOXALINDIONES, LEUR PREPARATION ET LEUR UTILISATION COMME ANTAGONISTES DES RECEPTEURS DE GLUTAMATE

(30) Priorität: 14.10.1994 DE 4436852
(43) Veröffentlichungstag der Anmeldung: 30.07.1997
(73) Patentinhaber: BASF AKTIENGESELLSCHAFT, 67056 Ludwigshafen (DE)
(72) Erfinder: LUBISCH, Wilfried, D-68159 Mannheim (DE); VIERLING, Michael, D-67125 Dannstadt-Schauernheim (DE); BEHL, Berthold, D-67117 Limburgerhof (DE); HOFMANN, Hans, Peter, D-67117 Limburgerhof (DE)
(86) Internationale Anmeldenummer: EP9503902
(87) Internationale Veröffentlichungsnummer: WO9611922

(56) Entgegenhaltungen:
- EP-A- 0 283 959
- EP-A- 0 374 534
- EP-A- 0 572 852

## Beschreibung

Die vorliegende Erfindung betrifft neue Pyrrolyl-tetrahydrobenzochinoxalindione, Verfahren zu deren Herstellung sowie deren Verwendung zur Bekämpfung von Krankheiten.

Die sogenannten exzitatorischen Aminosäuren, insbesondere Glutamat, sind im Zentralvervensystem weit verbreitet. Die exzitatorische Aminosäure Glutamat fungiert als Transmittersubstanz für Rezeptoren, von denen man verschiedene Subtypen kennt. Ein Subtyp wird z.B. nach dem spezifischen Agonisten N-Methyl-D-Aspartat NMDA-Rezeptor genannt. Dieser NMDA-Rezeptor weist verschiedene Bindungsstellen für Agonisten bzw. Antagonisten auf. Die Aminosäure Glycin bindet ebenfalls am NMDA-Rezeptor und moduliert die Wirkung des natürlichen Agonisten Glutaminsäure. Antagonisten an dieser Glycin-Bindungsstelle können danach antagonistische Effekte am NMDA-Rezeptor zeigen und eine "Überregung" dieses Rezeptors hemmen.

Zwei andere Subtypen der Glutamat-Rezeptoren stellen der AMPA- und der Kainat-Rezeptor dar, die jeweils nach den spezifischen Agonisten 2-Amino-3-hydroxy-5-methyl-4-isoxazole propionic acid (AMPA) und Kainic acid bezeichnet werden. Analog zum bereits genannten NMDA-Rezeptor könnten Antagonisten dieser Rezeptoren ebenfalls eine "Übererregung" hemmen.

Bei einer Reihe von neurodegenerativen Krankheiten oder psychischen Störungen treten erhöhte Glutamat-Spiegel auf, die zu Zuständen von Übererregungen oder toxischen Effekten im ZNS führen können.

Antagonisten gegen die Glutamat-Rezeptor-Subtypen können somit zur Behandlung dieser Krankheiten dienen. Glutamat-Antagonisten, dazu gehören insbesondere auch NMDA-Antagonisten bzw. deren Modulatoren (wie beispielsweise Glycin-Antagonisten) und die AMPA-Antagonisten, eignen sich zur therapeutischen Anwendung als Mittel gegen neurodegenerative Krankheiten (Chorea Huntington und Parkinsonsche Krankheiten), neurotoxische Störungen nach Hypoxie, Anoxie oder Ischämie, wie sie nach "Stroke" auftreten, oder auch als Antiepileptika, Antidepressiva und Anxiolytika (vgl. Arzneim. Forschung 1990, 40, 511 -514; TIPS, 1990, 11, 334 - 338 und Drugs of the Future 1989, 14 (11), 1059 - 1071).

Derivate des Chinoxalin-2,3(1H,4H)-dions II 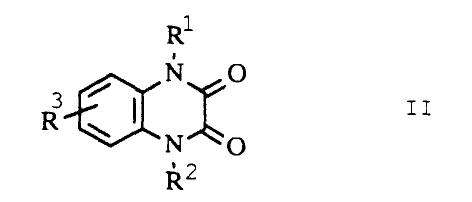 wurden bereits in mehreren Veröffentlichungen (EP 374.534 und EP 260.467) als Glutamat-Antagonisten beschrieben. Viele bekannte Derivate sind im heterocyclischen Chinoxalin-Fragment unsubstituiert (II, R¹, R² = Wasserstoff). Jedoch sind auch einige Derivate bekannt, bei denen R¹ in II nicht Wasserstoff ist. So sind in EP 377.112 und EP 374.534 N-Hydroxychinoxaline (II; R¹ = OR⁴) erwähnt worden. In EP 315.959, DE 4.135.871, WO 91/13.878 und WO 92/07.847 sind Alkylreste als R¹ in II beschrieben, wobei die Alkylkette noch durch Säuren, Ester oder Amide substituiert sein kann. Ebenfalls sind Alkylsäuren (= R¹) in Bioorg. & Med. Chemistry Lett. 1993, 3 (12), 2801-4 erwähnt.

N-Hydroxy-chinoxalindione (II, R¹ = OH) oder O-alkylierte Derivate sind bereits in EP 374.534 und EP 377.112 beschrieben worden. In EP 374.534 wurde auch ein N-Hydroxy-tetrahydrobenzochinoxalindion (Beispiel 5) synthetisiert. Unsubstituierte Tetrahydrobenzochinoxalindione (II, R¹ = R² = H) wurden in EP 283.959 beansprucht. Tetrahydrobenzochinoxalindione, die in R¹ oder R² einen substituierten Alkyl-Rest tragen sind bisher nie beschrieben worden.

Chinoxalindion-Derivate II, die einen Heterocyclus als Substituenten R³ tragen sind ebenfalls bekannt. So werden in EP 556.393 Imidazole, Triazole, Pyrazole erwähnt. Pyrrole (II, mit R³ = Pyrrolyl) sind in EP 572.852 als Glutamat-Antagonisten beschrieben worden.

Gegenstand der Erfindung sind neue Pyrrolyl-tetrahydrobenzochinoxalindione der Formel I 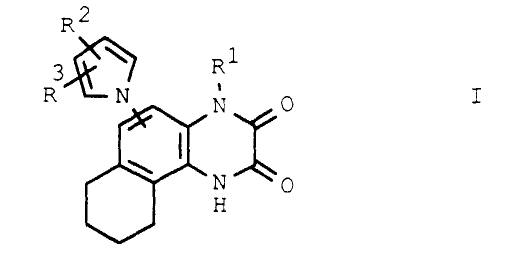 und ihre tautomeren und isomeren Formen, sowie ihre physiologisch verträglichen Salze, worin die Variablen folgende Bedeutung haben:
- R¹: Wasserstoff; ein aliphatischer Rest mit 1 oder 2 C-Atomen, der einen oder zwei verschiedene Substituenten der Formel-COOR⁴ tragen kann, worin R⁴ Wasserstoff oder C₁-C₄-Alkyl bedeutet;
-O-R⁶, worin R⁶ Wasserstoff oder eine CH₂-Gruppe ist, die einen der folgenden Reste tragen kann: -COOR⁴ oder Phenyl,
- R²: Wasserstoff, C₁-C₄-Alkyl oder Phenyl,
- R³: Wasserstoff oder der Rest -(CH₂)ₘ-R⁷, wobei m die Zahl 0, 1, 2, 3 oder 4 ist und R⁷ Wasserstoff, C₁-C₄-Alkyl, Phenyl, Phenylsulfonyl, NO₂, CN, -COO-(CH₂)ₙ-R⁸, -CONH-(CH₂)ₙ-R⁸, -CONHSO₂R⁴, -CO-R⁸, -CH=CH-CONHR⁸, -CH=CH-COOR⁸, -CH=NOR⁸, -CH₂-NR⁸R⁹, CH₂NH-CY-(CH₂)ₙR⁹, CH₂NH-CY-X-(CH₂)ₙ-R⁹, CH₂NH-CO-CF₃, CH₂NH-SO₂-R⁹ 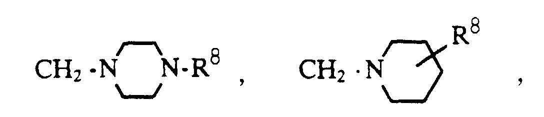 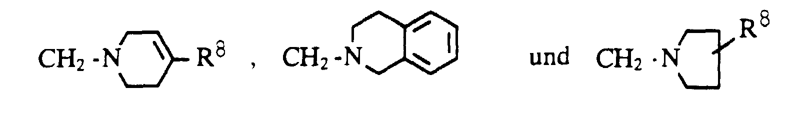 worin X und Y unabhängig voneinander Sauerstoff oder NH sind, n die Zahl 0, 1, 2, 3 oder 4 ist, R⁸ Wasserstoff oder geradliniges oder verzweigtes C₁-C₄-Alkyl bedeutet, das durch einen oder zwei Phenyl- oder Pyridyl-Reste substituiert sein kann, und R⁹ Wasserstoff, geradliniges oder verzweigtes C₁-C₆-Alkyl, Phenyl oder Pyridyl bedeutet, wobei alle in R⁸ und R⁹ enthaltenen Phenyl oder Pyridyl-Reste ein oder zwei der folgenden Reste tragen können: O-C₁-C₄-Alkyl, F, Cl, Br, J, C₁-C₄-Alkyl, NO₂, CF₃, -COOR⁵, -CONHR⁵, NH₂, CN, -SO₂Phenyl, -NHSO₂R⁵, -NHCOR⁵, OH, -SO₂-C₁-C₄-Alkyl, -NHCOCF₃, -SO₂R⁵ und -OCF₃.

Bevorzugt sind die Verbindungen der Formel I, ihre tautomeren und isomeren Formen, worin die Variablen folgende Bedeutung haben:
- R¹: Wasserstoff; ein aliphatischer Rest mit 1 oder 2 0-Atomen, der einen oder zwei verschiedene Substituenten der Formel -COOR⁴ tragen kann, worin R⁴ Wasserstoff oder C₁-C₄-Alkyl bedeutet,
-O-R⁶, worin R⁶ Wasserstoff oder eine CH₂-Gruppe ist, die einen der folgenden Reste tragen kann: -COOR⁴ oder Phenyl,
- R²: Wasserstoff
- R³: Wasserstoff oder der Rest -(CH₂)ₘ-R⁷, wobei m die Zahl 0 ist und R⁷ -COO-(CH₂)ₙ-R⁸, -CONH-(CH₂)ₙ-R⁸, -CO-R⁸, -CH₂-NR⁸R⁹, CH₂NH-CY-(CH₂)ₙR⁹, CH₂NH-CY-X- (CH₂)ₙR⁹, CH₂NH-CO-CF₃, CH₂NHSO₂R⁹ oder 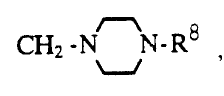 worin X und Y unabhängig voneinander Sauerstoff oder NH sind, n die Zahl 0, 1 oder 2 ist, R⁸ Wasserstoff oder geradliniges oder verzweigtes C₁-C₄-Alkyl bedeutet, das durch einen Phenyl-Rest substituiert sein kann, und R⁹ Wasserstoff, geradliniges oder verzweigtes C₁-C₆-Alkyl oder Phenyl bedeutet, wobei alle in R¹ und R⁹ enthaltener Phenyl-Reste ein oder zwei der folgenden Reste tragen können: O-C₁-C₄-Alkyl, F, Cl, C₁-C₄-Alkyl, NO₂, CF₃, -COOR⁵, -CONHR⁵, NH₂, CN, -SO₂Phenyl, -NHSO₂R⁵, -NHCOR⁵, OH, -SO₂-C₁-C₄-Alkyl, -NHCOCF₃, -SO₂R⁵ und -OCF₃ (R⁵ Wasserstoff, C₁-C₄-Alkyl, Phenyl oder Benzyl).

Die Darstellung der erfindungsgemäßen Verbindungen I kann auf verschiedenen Wegen erfolgen, wie die folgenden Reaktionsschemata zeigen.

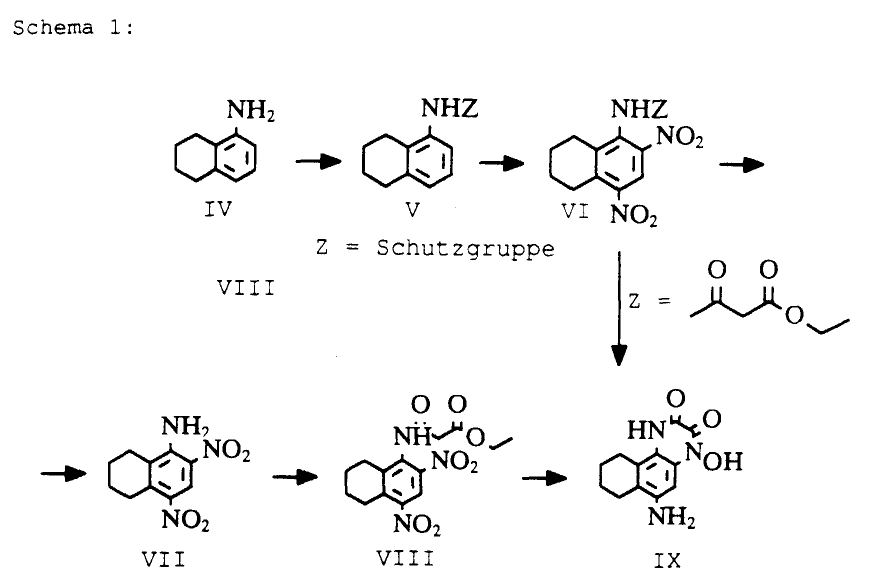

5-Aminotetralin (IV) wird in das gewünschte Derivat V überführt, wobei Z eine Schutzgruppe wie z.B. Acetyl und Trifluoracetyl darstellt. Weitere mögliche Schutzgruppen und Möglichkeiten zur Einführung sind in Th. W. Green und P. G. M. Wuts, "Protective Groups in Organic Synthesis", Wiley & Sons 1991, Kap. 7 aufgeführt. V wird analog üblicher Verfahren, die z.B. im Houben-Weyl-"Methoden zur organischen Chemie", Bd. 10/1 aufgelistet sind, nitriert. Dabei arbeitet man vornehmlich mit oder ohne Lösungsmittel wie Schwefelsäure und Essigsäure mit Nitrierungsmitteln wie Kaliumnitrat und Salpetersäure bei Temperaturen von 0 - 50°C, bevorzugt bei 0 - 25°C.

Wenn Z = -CO-COOEt ist, kann VI direkt zum Chinoxalindion IX reduziert werden. Diese Reduktion wird vorzugsweise katalytisch mit Wasserstoff in polaren Lösungsmitteln wie Alkohole und Dimethylformamid durchgeführt. Als Katalysatoren können z.B. Palladium/Kohle oder Platin/Kohle eingesetzt werden.

In anderen Fällen wird die Schutzgruppe Z in VI hydrolytisch mit Säuren, z.B. Salzsäure, oder Basen, z.B. Natronlauge, bei Temperaturen von 25 - 100°C abgespalten. Das so erhaltene Anilin wird mit Oxalsäure-Derivaten zum Oxalanilid VIII umgesetzt. Diese Amidbildung erfolgt nach üblichen Verfahren, die z.B. im Houben-Weyl, "Methoden der organischen Chemie" Bd. E 5, Kap. V, aufgeführt sind. Die Reduktion mit nachfolgendem Ringschluß von XIII zum Chinoxalindion IX ist oben beschrieben worden.

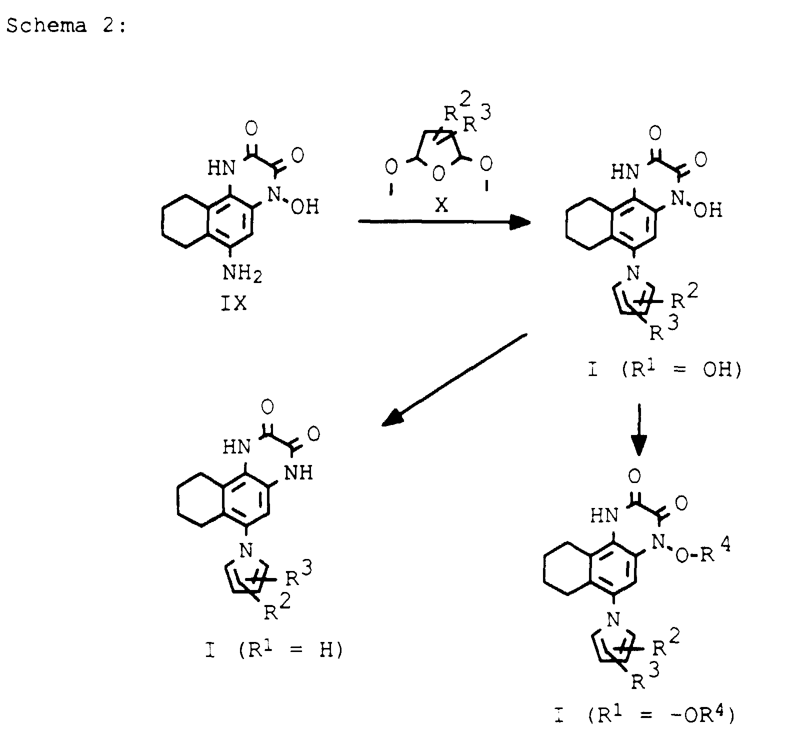

Das Anilin IX wird mit einer 1,4-Dicarbonyl-Verbindungen X wie Bernsteindialdehyd-Derivate oder davon abgeleiteten cyclischen oder acyclischen Acetalen zu Pyrrolen I (R¹ = OH) umgesetzt. Dabei arbeitet man nach üblichen Verfahren, die z.B. von C. Ferri in Reaktionen der organischen Synthese, Thieme-Verlag 1978, S. 708f. beschrieben sind und die weiter unten ausführlicher diskutiert werden.
Das Pyrrol-Derivat I (R¹ = OH) kann danach zu den analogen Verbindungen I (R¹ = H) reduziert werden. Diese Reduktion erfolgt ebenso nach üblichen Methoden, bevorzugt aber mit Eisen in Essigsäure bei Temperaturen von 50 - 120°C.

Ebenso kann das Pyrrol-Derivat I (R¹ = OH) an der Hydroxy-Gruppe zu I (R¹ = -OR⁴) mit R⁴-Halogen alkyliert werden. Diese Reaktion wird in polaren Lösungsmitteln wie Dimethylformamid, Alkoholen, Wasser oder Gemischen daraus durchgeführt und als Basen werden, je nach Lösungsmittel z.B. Alkoholate, Karbonate und Hydrogenphosphate eingesetzt. Die Reaktion wird bei Temperaturen von 0 - 70°C, bevorzugt bei Raumtemperatur, durchgeführt.

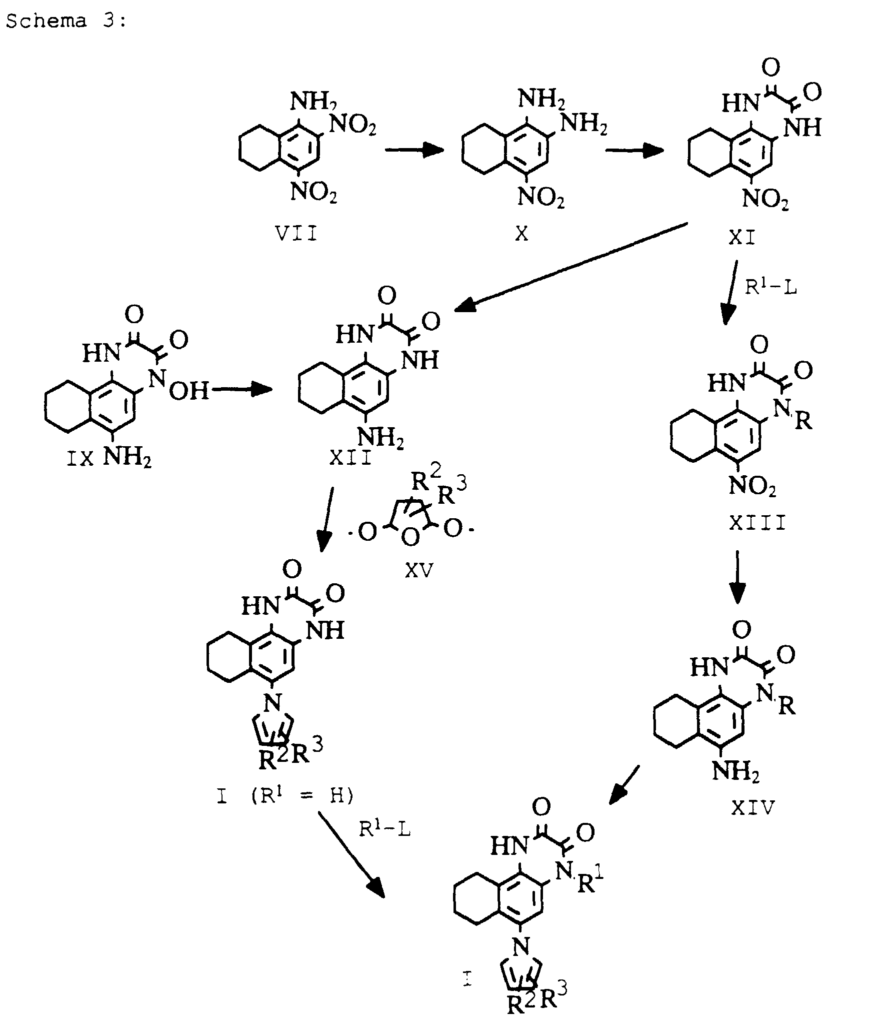

Die Verbindung VII (Schema 1) kann auch zum ortho-Diamino-Derivat X reduziert werden. Diese Reduktion gelingt vornehmlich mit Schwefel oder Schwefelverbindungen, wie Alkalisulfiden, -polysulfiden oder analogen Ammoniumverbindungen. Man arbeitet häufig in wäßrigen Medien bei alkalischen pH-Werten und erhöhten Temperaturen bis 100°C. Ausführlicher sind diese Reduktionsmethoden im Houben-Weyl, "Methoden zur organischen Chemie, Bd. 11/1, Kap. IV beschrieben.

Das Diamin X kann anschließend mit Oxalsäure-Derivaten zum Chinoxalindion XI umgesetzt werden. Im Falle des Oxalsäurediesters wird die Verbindung X mit dem Diester ohne Lösungsmittel z.B. unter Rückfluß erwärmt, wobei das Produkt entsteht. Setzt man das Oxalsäuremonochlorid ein, arbeitet man wie bei Amid-Synthesen (siehe R. C. Larock, Comprehensive Organic Chemistry, Kap. 9,4)) und erwärmt das erhaltene Monoamid anschließend mit oder ohne Lösungsmittel, wobei das Produkt XI entsteht. Erhält man dabei das Diamid von X, wird dies in wäßrigen Säuren wie Salzsäure unter Zusatz von Lösungsvermittlern wie Tetrahydrofuran erwärmt, wobei das Chinoxalin-Derivat XI anfällt. Die Nitrogruppe in XI kann anschließend reduziert werden, und man erhält das Anilin XII.

Diese Reduktion kann nach chemischen und nach katalytischen Varianten erfolgen. Bei der katalytischen Methode wird z.B. mit Wasserstoff an Katalysatoren wie z.B. Palladium/Kohle und Platin/ Kohle in Lösungsmitteln wie Alkoholen, Tetrahydrofuran oder Dimethylformamid gearbeitet, wobei aber auch chemische Substanzen wie Ammoniumformiat als Wasserstoffüberträger eingesetzt werden können. Auf chemischem Wege erfolgt die Reduktion mit Metallen oder Metallsalzen wie Eisen und Zinn in Gegenwart von Säuren wie Salzsäure und Essigsäure bei in der Regel erhöhter Temperatur, z.B. 60 - 120°C. Weitere Reduktionsmöglichkeiten sind in Houben-Weyl, Methoden der organischen Chemie, Bd. 11/1, Kap. IV aufgeführt.

Das Anilin XII kann ebenfalls aus dem N-Hydroxy-chinoxalindion IX durch chemische Reduktion (s. oben) hergestellt werden. XII wird nach einer Paal-Knorr-Methode mit 1,4-Dicarbonylverbindungen zum Pyrrol I (R¹ = H) umgesetzt. Dies geschieht in üblicher Weise, wie es z.B. in C. Ferri, "Reaktionen der organischen Synthese", Thieme-Verlag, 1978, S. 708 f. beschrieben ist. Als Reaktionskomponente dienen 1,4-Dicarbonylverbindungen wie Aldehyde, Ketone, Ketoaldehyde oder deren Acetale, die wie in XV auch cyclisch sein können. Man arbeitet in Lösungsmitteln in Gegenwart von katalytischen Mengen Säuren, wie Essigsäure oder Toluolsulfonsäure unter Wasserabspaltung. Die Säure kann auch als Lösungsmittel dienen, wenn sie in großen Mengen verwendet wird. Im allgemeinen wird die umsetzung jedoch in Lösungsmitteln wie Toluol oder in einem Lösungsmittelgemisch wie Toluol/Dimethylformamid unter Säurekatalyse bei einer Reaktionstemperatur von 50 - 150°C, vorzugsweise 100 -150°C, oder in konzentrierter Essigsäure bei Temperaturen von 50°C bis zum Siedepunkt ausgeführt.

Das Chinoxalin I (R¹ = H) in Schema 3 kann danach mit einer Verbindung R¹-L zu I alkyliert werden, wobei L für eine Abgangs- oder "Leaving"-Gruppe steht und z.B. Halogenide (Chlor, Brom, Jod), Triflate und Tosylate sein kann. Diese Alkylierung wird in polaren, aprotischen Lösungsmitteln wie Tetrahydrofuran und Dimethylformamid bei Temperaturen von - 10 bis 100°C ausgeführt, wobei das Chinoxalindion I (R¹ = H) zuerst mit Basen wie Natriumhydrid oder Kalium-tert.-butylat deprotoniert und anschließend R¹-L zugefügt wird.

Alternativ kann diese Stufensequenz auch vertauscht werden, wobei XI zuerst alkyliert wird und das so erhaltene XIII über Reduktion zu XIV ins Pyrrol I überführt wird (s. Schema 3).

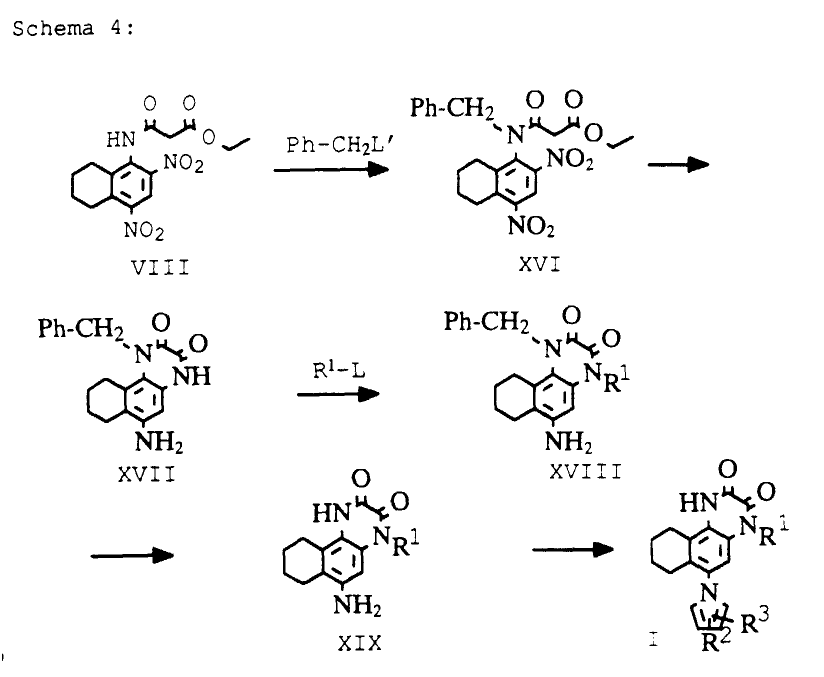

Eine weitere Synthese ist im Schema 4 dargestellt. Das Oxalamid VIII wird zum Derivat XVI alkyliert. Dabei arbeitet man in polaren, aprotischen Lösungsmitteln wie Tetrahydrofuran und Dimethylformamid, wobei man VIII zuerst mit Basen wie z.B. Natriumhydrid oder Kaliumtert.-butanolat deprotoniert und anschließend das Alkylierungsreagenz PhCH₂L, zugibt, wobei L' eine Abgangsgruppe wie L (siehe Schema 3) sein kann. Diese Reaktion wird bei Temperaturen von 0 bis 100°C durchgeführt.

Die Dinitroverbindung XVI wird anschließend zum Chinoxalindion XVII reduziert. Diese Reduktion wird analog Schema 1 und 2 durchgeführt, wobei hier bevorzugt in Eisessig mit Eisen bei 100°C bis Siedetemperatur gearbeitet wird. XVII wird wie in Schema 3 (Synthese von I bzw. XIII) mit R¹-L alkyliert, wobei L Abgangsgruppen wie z.B. Halogenide darstellt.

Das Chinoxalindion XVIII wird danach durch katalytische Hydrierung in das Derivat XIX überführt. Diese katalytische Hydrierung wird wie bereits zuvor beschrieben in Lösungsmitteln wie z.B. Tetrahydrofuran, Alkoholen und Dimethylformamid mit Wasserstoff oder Wasserstoffüberträgern wie Ammoniumformiat in Gegenwart eines Katalysators wie z.B. Palladium/Kohle oder Platin/Kohle ausgeführt.

Das Anilin XIX reagiert anschließend mit einer 1,4-Dicarbonylverbindung oder einem Derivat davon in einer Paal-Knorr-Synthese, analog Schema 3, zu dem erfindungsgemäßen Pyrrol I.

In den so hergestellten Produkten Ia kann die Substitution des beanspruchten Pyrrolyl-Ringes in geeigneter Weise abgewandelt werden (Schema 5). So kann der Aldehyd durch reduktive Aminierung mit Aminen in die erfindungsgemäßen Verbindungen Ib überführt werden. Die reduktive Aminierung wird im allgemeinen bei Temperaturen von 5 bis 80°C, vorzugsweise 10 bis 30°C, in Gegenwart von Reduktionsmitteln wie Natriumcyanoborhydrid oder Wasserstoff in Gegenwart eines Hydrierkatalysators wie Pd/Kohle, Pt/Kohle oder Raney-Nickel, zweckmäßig in polaren organischen Lösungsmitteln wie Alkoholen oder Dimethylformamid, durchgeführt.

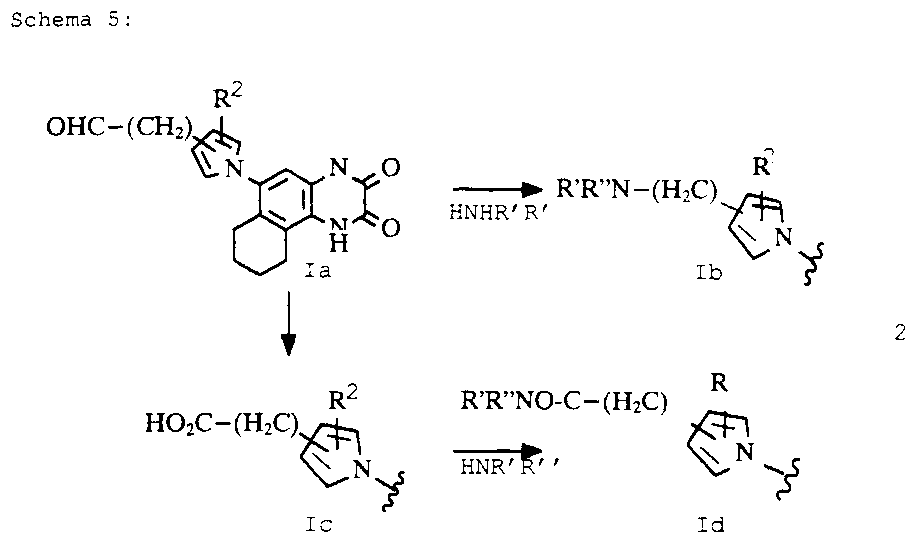

Der Aldehyd Ia kann nach üblichen Verfahren, die z.B. in R. C. Larock, "Comprehensive Organic Transformations", 1989, VCH Publisher, S. 838 f., zur erfindungsgemäßen Carbonsäure Ic oxidiert werden, insbesondere mit Kaliumpermanganat in Lösungsmitteln wie Aceton bei Temperaturen von 25 - 60°C. Diese Carbonsäuren Ic werden durch Reaktion mit Aminen NHR'R" in die Amide Id überführt. Die Kupplung geschieht nach bekannten Verfahren, die z.B. im Houben-Weyl, "Methoden der organischen Chemie", Band E5, Kapitel V, aufgelistet sind.

Die Pyrrolylalkylamine können ebenfalls mit Isocyanaten zu den Harnstoffen Ig umgesetzt werden, wobei man anstelle der Isocyanate auch Amine HNR'R" verwenden kann, die in bekannter Weise zuvor mit Phosgen oder analogen Verbindungen, wie Carbonyldiimidazol (= CDI), umgesetzt werden. Diese und vergleichbare Verfahren sind beispielsweise in Houben-Weyl "Methoden der organischen Chemie", Band E4, S. 334 ff. beschrieben. Bei diesen Verfahren arbeitet man mit oder onne Lösungsmittel, bevorzugt Dimethylformamid, und bei Temperaturen von 25 - 150°C.

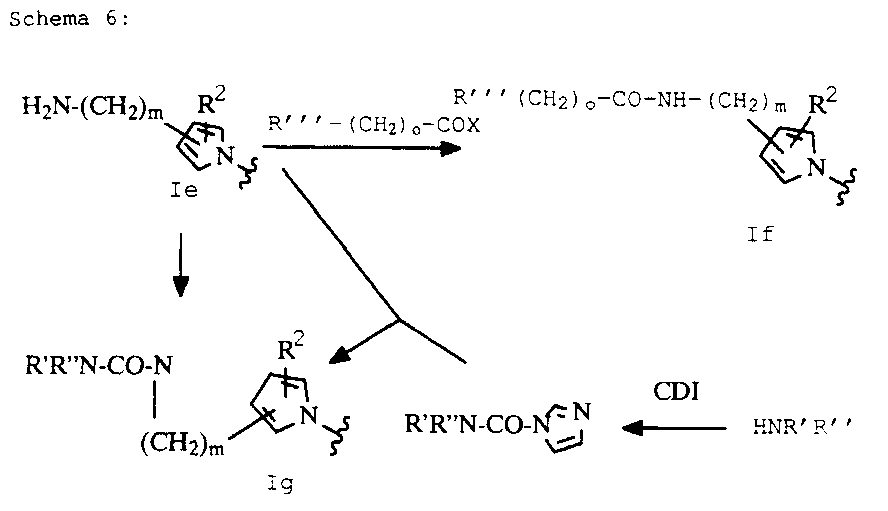

Die nach Schema 5 zugänglichen Pyrrolylalkylamine Ie können mit Säuren R''' -(CH₂)ₒCO₂H, die in geeigneter Weise zu R''' (CH₂)ₒCOL" aktiviert werden, wobei L" für eine Abgangsgruppe wie Azid, Imidazol und andere, die bei R. C. Larock, Comprehensive Organic Transformations, New York 1989, S. 972 ff, aufgelistet sind, in die erfindungsgemäßen Amide If überführt werden. Diese Kupplung geschieht nach bekannten Verfahren, die z.B. im Houben-Weyl "Methoden der organischen Chemie", Band E5, Kapitel V, aufgelistet sind.

Die erfindungsgemäßen Verbindungen stellen Antagonisten der exzitatorischen Aminosäure Glutamat,insbesondere Antagonisten der Glycin-Bindungsseite des NMDA-Rezeptors, des AMPA-Rezeptors und des Kainat-Rezeptors dar.

Die pharmakologische Wirksamkeit der Verbindungen I wurde an isoliertem Membranmaterial von Rattengroßhirnen untersucht. Hierzu wurde das Membranmaterial in Gegenwart der erfindungsgemäßen Verbindungen mit den radioaktiv markierten Substanzen ³H-2-Amino-3-hydroxy-5-methyl-4-isoxazolpropionsäure (³H-AMPA), [³H]-Glycin oder [³H]-Kainate behandelt, wobei sich diese an spezifischen Rezeptoren (AMPA-, NMDA- oder Kainat-Rezeptoren) binden. Anschließend wurde durch Scintillationszählung die Radioaktivität der behandelten Membrane gemessen. Über die gebundene Radioaktivität ließen sich die Mengen an gebundener ³H-AMPA, [³H]-Glycin oder [³H]-Kainat bzw. jeweils die verdrängten Mengen dieser radioaktiv markierten Substanzen bestimmen. Die sich hieraus ergebende Dissoziationskonstante K_{I} (I = Inhibitor), welche ein Maß für die Verdrängungswirkung des erfindungsgemäßen Wirkstoffes ist, wurde durch iterative nichtlineare Regressionsanalyse mit dem Statistical Analysis System (SAS) an einem IBM-Rechner, ähnlich dem Programm "Ligand" von P. J. Munson und D. Rodbard (Analytical Biochem. 107, 220 (1980), Ligand: Versatile Computerized Approach for Charakterization of Ligand Binding Systems) ermittelt.

Folgende In-Vitro-Untersuchungen wurden durchgeführt:
1. Bindung von ³H-2-Amino-3-hydroxy-5-methyl-4-isoxazolpropionsäure (³H-AMPA)
   Für die Präparation des Membranmaterials wurden frisch entnommene Rattengroßhirne zusammen mit dem 15fachen Volumen einer Pufferlösung A aus 30 mM α,α,α-Tris-(hydroxymethyl)methylamin-Hydrochlorid (TRIS-HCl) und 0,5 mM Ethylendiamintetraessigsäure (EDTA) - pH 7,4 - mittels eines Ultra-Turrax® -Rührers homogenisiert. Die Suspension wurde 20 min bei 48000 x g zentrifugiert. Nach Abtrennung der überstehenden Flüssigkeit wurde das im Bodensatz enthaltene proteinhaltige Membranmaterial dreimal durch Suspendieren in der Pufferlösung A und anschließendes, jeweils 20minütiges Zentrifugieren bei 48000 x g gewaschen. Danach wurde das Membranmaterial in einem 15fachen Volumen der Pufferlösung A suspendiert und 30 min bei 37°C inkubiert. Anschließend wurde das Proteinmaterial zweimal durch Zentrifugieren und Suspendieren gewaschen und bis zur Verwendung bei - 70°C eingefroren.
   Für den Bindungstest wurde das bei 37°C aufgetaute Proteinmaterial zweimal durch Zentrifugieren bei 48000 x g (20 min) und anschließendes Suspendieren in einer Pufferlösung B aus 50 mM TRIS-HCl, 0,1 M Kaliumthiocyanat und 2,5 mM Calciumchlorid - pH 7,1 - gewaschen. Anschließend wurden 0,25 mg Membranmaterial, 0,1 µCi ³H-AMPA (60 Ci/mmol) sowie Verbindung I und 1 ml Pulverlösung B gelöst und 60 min auf Eis inkubiert. Die inkubierte Lösung wurde über einen CF/B-Filter (Firma Whatman), der zuvor mindestens 2 h mit einer 0,5 %igen wäßrigen Lösung von Polyethylenimin behandelt worden war, filtriert. Anschließend wurde der Membranrückstand mit 5 ml kalter Pufferlösung B gewaschen, um gebundene und freie ³H-AMPA voneinander zu trennen. Nach Messung der Radioaktivität der gebundenen ³H-AMPA im Membranmaterial durch Scintillationszählung wurde durch Auswertung der Verdrängungskurven mittels Regressionsanalyse der K_{I}-Wert bestimmt.
   Für 9-(1-Pyrrolyl)-5,6,7,8-tetrahydrobenzo[f]chinoxalin-2,3-(1H,4H)-dion (Beispiel 3) wurde ein K_{I}-Wert von <10 µM ermittelt. Die Substanz ist wirksamer als die verwandten Substanzen des Beispiels 20 aus EP 573221 und des Beispiels 16 aus EP 283.959.
2. Bindung von [³H]-Glycin
   Für die Präparation der Membranen für den ³H-Glycin-Bindungsassay wurde frisch entnommene Rattenhippocampi in 10fachem Volumen Präparationspuffer (50 mM Tris-HCl, 10 mM EDTA) mit einem Potter-Homogenisator homogenisiert. Das Homogenat wurde 20 min bei 48000 x g zentrifugiert. Der Überstand wurde verworfen und die im Pellet erhaltenen Membranen durch Resuspendieren und Zentrifugieren bei 48000 x g (jeweils 20 min) 2 x gewaschen. Die resuspendierten Membranen wurden in flüssigem Stickstoff eingefroren und bei 37°C wieder aufgetaut. Nach einem erneuten Waschschritt wurde die Membransuspension 15 min bei 37°C im Schüttelwasserbad inkubiert. Nach 4 weiteren Waschschritten (jeweils 20minütiges Zentrifugieren bei 48000 x g und Resuspendieren in Präparationspuffer) wurden die Membranen bis zur weiteren Verwendung bei - 70°C eingefroren.
   Die eingefrorenen Membranen wurde bei 37°C aufgetaut und 2 x durch Zentrifugation bei 48000 x g (20 min) und anschließendes Resuspendieren in Bindungspuffer (50 mM Tris-HCl pH 7,4, 10 mM MgCl₂) gewaschen. Ein Inkubationsansatz enthielt 0,25 mg Protein (Membranen), 25 nM ³H-Glycin (16 Ci/mMol) und die zu testenden Substanzen in insgesamt 0,5 ml Bindungspuffer. Die unspezifische Bindung wurde durch Zugabe von 1 mM Glycin bestimmt. Nach 60 min Inkubation bei 4°C wurde gebundener und freier Ligand durch Filtration über GF/B-Filter und anschließendem Waschen mit ca. 5 ml eiskaltem Bindungspuffer voneinander getrennt. Die auf den Filtern verbleibende Radioaktivität wird durch Flüssgkeitsscintillationszählung bestimmt. Aus den Verdrängungskurven wurden mit Hilfe eines iterativen nichtlinearen Anpassungsprogramms oder entsprechend der Gleichung von Cheng und Prusoff die Dissoziationskonstanten berechnet.
3. Bindung von [³H]-Kainat
   Für die Präparation der Membranen für den [³H]-Kainat-Bindungsassay wurden frisch entnommene Großhirne von Ratten in Präparationspuffer (30 mM Tris-HCl pH 7,4, 0,5 mM EDTA) mit Hilfe eines Ultra-Turrax^{R} in 15fachem Volumen homogenisiert. Das Homogenat wurde bei 48000 x g 20 min zentrifugiert. Der Überstand wurde verworfen und die im Pellet enthaltenen Membranen durch Resuspendieren in Präparationspuffer und Zentrifugieren bei 48000 x g (jeweils 20 min) insgesamt 3 x gewaschen. Nach dem dritten Waschschritt wurden die Membranen 2 x durch Zentrifugation und Resuspension gewaschen und bis zur weiteren Verwendung bei - 70°C eingefroren.
   Die eingefrorenen Membranen wurden bei 37°C aufgetaut, in Bindungspuffer (50 mM Tris-HCl pH 7,4) suspendiert und 20 min bei 48000 x g zentrifugiert. Die sich im Pellet befindlichen Membranen wurden erneut in Bindungspuffer resuspendiert. Ein Inkubationsansatz enthielt 0,25 mg Protein (Membranen), 0,058 µCi (58 Ci/mmol) sowie die zu testenden Substanzen in insgesamt 1 ml Bindungspuffer. Die unspezifische Bindung wurde in Gegenwart von 0,1 mM Glutamat bestimmt. Nach erfolgter 60minütiger Inkubation auf Eis wurde gebundener und freier Ligand durch Filtration über CF/B-Filter und anschließendes Waschen mit 5 ml eiskaltem Bindungspuffer voneinander getrennt. Die CF/B-Filter waren zuvor für mindestens 2 h mit 0,5 % Polyethylenimin behandelt worden. Die Auswertung der Verdrängungskurven bzw. Berechnung der Dissoziationskonstanten erfolgte durch ein nicht-lineares Anpassungsprogramm oder entsprechend der Gleichung von Cheng und Prusoff.
   Die erfindungsgemäßen Verbindungen I sind als Arzneimittel für die Human- und Veterinärmedizin geeignet und können zur Herstellung von Arzneimitteln zur Behandlung neurodegenerativer Erkrankungen wie Morbus Parkinson und Chorea Huntington und neurotoxischer Störungen des zentralen Nervensystems wie cerebraler apoplektischer Insulte (z.B. "Stroke") und traumatischer Läsionen des Gehirns und des Rückenmarks sowie zur Herstellung von Antiepileptika, Anxiolytika und Antidepressiva verwendet werden.
   Die erfindungsgemäßen Arzneimittelzubereitungen enthalten neben den üblichen Arzneimittelhilfsstoffen eine therapeutisch wirksame Menge der Verbindungen I. Für die lokale äußere Anwendung, z.B. in Puder und Salben, können die Wirkstoffe in den üblichen Konzentrationen enthalten sein. In der Regel sind die Wirkstoffe in einer Menge von 0,0001 bis 1 Gew.-%, vorzugsweise 0,001 bis 0,1 Gew.-%, enthalten.
   Bei der inneren Anwendung werden die Präparationen in Einzeldosen verabreicht. In einer Einzeldosis werden pro kg Körpergewicht 0,1 bis 100 mg gegeben. Die Zubereitungen können täglich in einer oder mehreren Dosierungen je nach Art und Schwere der Erkrankungen verabreicht werden.
   Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes hydriertes Ricinusöl, Polyacrylsäure, Polyetylenglykol, Polyethylenglykostearat, ethoxylierte Fettalkohole, Paraffinöl, Vaseline und Wollfett verwendet werden. Für die innere Anwendung eignen sich z.B. Milchzucker, Propylenglykol, Ethanol, Stärke, Talk und Polyvinylpyrrolidon.
   Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.
   Die neben dem Wirkstoff in der Zubereitung enthaltenen Stoffe sowie die bei der Herstellung der pharmazeutischen Zubereitung verwendeten Stoffe sind toxikologisch unbedenklich und mit dem jeweiligen Wirkstoff verträglich. Die Herstellung der Arzneimittelzubereitungen erfolgt in üblicher Weise, z.B. durch Vermischen des Wirkstoffes mit den anderen üblichen Trägerstoffen und Verdünnungsmitteln.
   Die Arzneimittelzubereitungen können in verschiedenen Applikationsweisen verabreicht werden, wie peroral, parenteral, subkutan, intraperitoneal und topisch. So sind Zubereitungsformen wie Tabletten, Emulsionen, Infusions- und Injektionslösungen Pasten, Salben, Gele, Cremes, Lotionen, Puder und Sprays möglich.

### Beispiele

### Beispiel 1

9-(3-Formyl-1-pyrrolyl)-1-hydroxy-5,6,7,8-tetrahydrobenzo[f]-chinoxalin-2,3(1H,4H)-dion 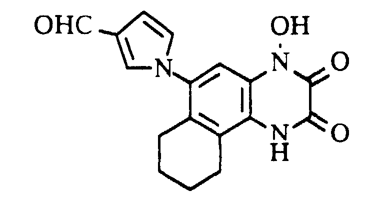
a) Oxalsäuremonoethylester-(5,6,7,8-tetrahydronaphthyl-1)-amid
   100 g (0,68 Mol) 5,6,7,8-Tetrahydronaphthyl-1-amin und 188 ml (1,36 Mol) Triethylamin wurden in 1,5 L wasserfreiem Tetrahydrofuran gelöst und bei 0 - 5°C mit 102,5 g (0,75 Mol) Oxalsäuremonoethylesterchlorid tropfenweise versetzt. Danach wurde noch 30 min gerührt. Der ausgefallene Niederschlag wurde abgesaugt und das Filtrat im Vakuum eingeengt. Der Rückstand wurde aus Ethanol umkristallisiert. Man erhielt 159 g (95 %) Produkt.
   ¹H-NMR (D₆-DMSO): δ = 1,3 (3H); 1,7 (4H); 2,6 (2H); 2,8 (2H); 4,3 (2H), 6,9 - 7,3 (3H) und 10,2 (1H) ppm.
b) Oxalsäuremonoethylester-(2,4-dinitro-5,6,7,8-tetrahydronaphthyl-1)-amid
   159 g (0,64 Mol) des Produktes 1a wurden in 1,5 L konzentrierter Schwefelsäure gelöst. Bei ca. 10°C tropfte man 83 ml 98%ige Salpetersäure langsam zu und rührte 30 min. Anschließend wurde der Reaktionsansatz vorsichtig auf viel Eis gegossen und der Niederschlag abgesaugt. Man erhielt 122 g (56 %) Produkt.
   ¹H-NMR (D₆-DMSO): δ = 1,3 (3H); 1,7 (4H); 2,8 (2H); 3,0 (2H); 4,3 (2H); 8,4 (1H) und 11 (1H) ppm.
c) 9-Amino-1-hydroxy-5,6,7,8-tetrahydrobenzo[f]chinoxalin-2,3-(1H,4H)-dion
   120 g (0,36 Mol) des Produktes 1b wurden in 2 L Tetrahydrofuran gelöst und nach Zugabe von 5 g Pd/Kohle (10 %) hydriert. Danach wurde filtriert und der Niederschlag gut mit Dimethylformamid ausgekocht. Die vereinigten organischen Phasen wurden im Vakuum eingeengt und der Rückstand mit Ethanol behandelt. Der anfallende Niederschlag wurde abgesaugt. Man erhielt 57 g (65 %) Produkt.
   ¹H-NMR (D₆-DMSO): δ = 1,7 (4H); 2,3 (2H); 2,7 (2H); ca. 5 (breit, NH₂); 6,8 (1H); 11,0 (1H) und 11,5 (breit) ppm.
d) 9-(3-Formyl-1-pyrrolyl)-1-hydroxy-5,6,7,8-tetrahydrobenzo[f]-chinoxalin-2,3(1H,4H)-dion
   2,4 g (9,7 mMol) des Produktes 1c und 1,5 g (9,7 mMol) 2,5-Dimethoxytetrahydrofuran-1-ylcarbaldehyd wurden in 100 ml Eisessig 30 min unter Rückfluß gekocht. Danach wurde alles im Vakuum eingeengt. Der Rückstand wurde mit Ethanol und anschließend mit heißem Tetrahydrofuran behandelt und abgesaugt. Man erhielt 2,3 g (74 %) Produkt, Schmp. > 250°C.
   ¹H-NMR (D₆-DMSO) : δ = 1,6 (2H): 1,8 (2H); 2,5 (2H); 2,8 (2H); 6,6 (1H); 7,0 (1H); 7,3 (1h); 7,8 (1H); 9,8 (1H) und ca. 11,3 (breit) ppm.

### Beispiel 2

9-(2,5-Dimethyl-1-pyrrolyl)-1-hydroxy-5,6,7,8-tetrahydrobenzo[f]-chinoxalin-2,3(1H,4H)-dion

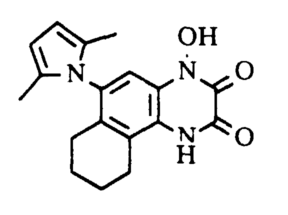

8 g (32,2 mMol) des Produktes 1c und 3,8 ml (32,3 mMol) Hexan-2,5dion wurden analog Vorschrift 1e umgesetzt. Man erhielt 7,2 g (69 %) Produkt, Schmp. > 250°C.

¹H-NMR (D₆-DMSO): δ =1,6 (2H); 1,8 (2H); 1,9 (6H); 2,1 (2H); 2,9 (1H); 5,8 (2H); 7,1 (1H); 11,4 (1H) und ca. 11,8 (breit) ppm.

### Beispiel 3

1-Hydroxy-9-(1-pyrrolyl)-5,6,7,8-tetrahydrobenzo[f]chinoxalin2,3-(1H,4H)-dion

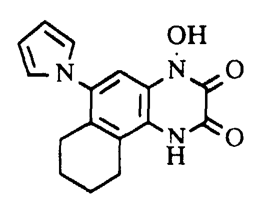

1,2 g (4,8 mMol) des Produktes 1c und 0,61 g (4,8 mMol) 2,5-Dimethoxytetrahydrofuran wurden analog Vorschrift 1e umgesetzt. Man erhielt 1,15 g (82 %) Produkt, Schmp. > 250°C.

¹H-NMR (D₆-DMSO) : δ = 1,6 (2H); 1,8 (1H); 2,5 (2H); 2,8 (1H); 6,2 (2H); 6,8 (2H); 7,2 (1H); 11,4 (1H) und ca. 12 (breit) ppm.

### Beispiel 4

1-Hydroxy-9-(3-trifluormethylamidomethyl-1-pyrrolyl)-5,6,7,8-tetra-hydrobenzo[f]chinoxalin-2,3(1H,4H)-dion 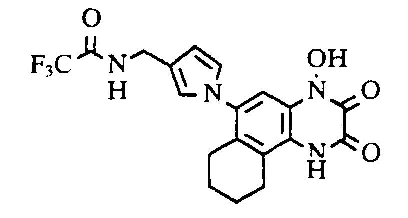
a) Herstellung von N-((2,5-Dimethoxy-tetrahydrofuran-3-yl)-methyl)-trifluoressigsäureamid
   50 g (0,31 Mol) 3-Aminomethyl-2,5-dimethoxytetrahydrofuran (DE 2.645.234), 31,7 g (0,31 Mol) Triethylamin und etwas 4-(N,N-Dimethylamino)pyridin wurden in 300 ml wasserfreiem Ether gelöst und bei 0 bis 5°C tropfenweise mit 65,1 g (0,31Mol) Triflucressigsäureanhydrid, das in 100 ml wasserfreiem Ether gelöst war, versetzt. Man rührte noch 1 h. Danach wurde der Ansatz mit Wasser gewaschen, getrocknet und im Vakuum eingeengt. Man erhielt 70,5 g verunreinigtes Produkt, das ohne Reinigung weiter umgesetzt wurde.
b) 1-Hydroxy-9-(3-trifluormethylamidomethyl-1-pyrrolyl)-5,6,7,8-tetrahydrobenzo[f]chinoxalin-2,3(1H,4H)-dion
   3 g (12,1 mMol) des Produktes 1c und 3,1 g (12,1 mMol) des Produktes 4a wurden analog Vorschrift 1d umgesetzt. Man erhielt 3,9 g (77 %) Produkt, Schmp. > 230°C.
   ¹H-NMR (D₆-DMSO): δ = 1,6 (2H); 1,8 (2H); 2,4 (2H); 2,8 (2H); 4,3 (2H); 6,2 (1H); 6,9 (2H); 7,2 (1H); 9,8 (1H); 11,4 (1H) und ca. 12 (breit) ppm.

### Beispiel 5

9-(3-Aminomethyl-1-pyrrolyl)-1-hydroxy-5,6,7,8-tetrahydrobenzo-[f]-chinoxalin-2,3(1H,4H)-dion

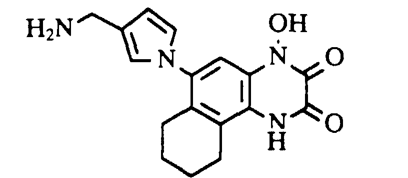

3,6 g (8,5 mMol) des Beispiels 4 wurden in 30 ml Tetrahydrofuran gelöst und mit 0,6 g (25,6 mMol) Lithiumhydroxid, gelöst in 50 ml Wasser, versetzt. Man rührte etwa 2 h bei Raumtemperatur. Danach wurde das Tetrahydrofuran im Vakuum entfernt und die resultierende Wasserphase mit verdünnter Salzsäure leicht angesäuert. Nach Zugabe von wäßriger Natriumhydrogenkarbonat-Lösung fiel das Produkt aus (pH < 7) und wurde abgesaugt. Man erhielt 2,9 g (100 %), Schmp. > 250°C.

¹H-NMR (CD₃COOD): δ = 1,7 (2H); 1,9 (2H); 2,5 (2H); 2,9 (2H); 4,2 (2H); 6,4 (1H); 6,8 (1H); 7,0 (1H) und 7,5 (1H) ppm.

### Beispiel 6

N-(1-(1-Hydroxy-5,6,7,8-tetrahydrobenzo[f]chinoxalin-2,3(1H,4H)-dion-9-yl)pyrrol-3-yl)methyl-N'-phenyl-harnstoff

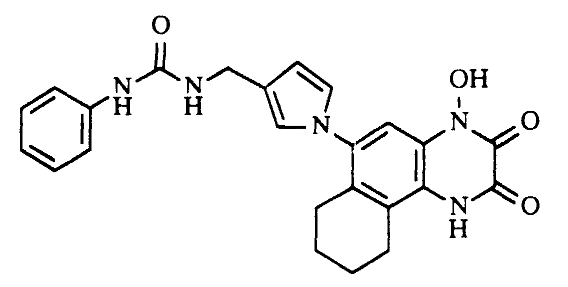

0,75 g (2,3 mMol) Substanz des Beispieis 5 und 0,37 g (3,1 mMol) Phenylisocyanat wurden in 30 ml wasserfreiem Dimethylformamid für 25 min auf 100°C erhitzt. Anschließend wurde im Vakuum eingeengt. Der Rückstand wurde in Ethanol dispergiert und abgesaugt. Man erhielt 0,8 g (79 %) Produkt, Schmp. > 250°C.

¹H-NMR (D₆-DMSO): δ = 1,7 (2H); 1,8 (2H); 2,5 (2H); 2,8 (2H); 4,2 (2H); 6,2 (1H); 6,3 (1H); 6,8 - 7,6 (8H); 8,4 (1H); 11,4 (1H) und ca. 12,0 (breit) ppm.

### Beispiel 7

9-(3-Benzoylaminomethyl-1-pyrrolyl)-1-hydroxy-5,6,7,8-tetrahydrobenzo[f]chinoxalin-2,3(1H,4H)-dion 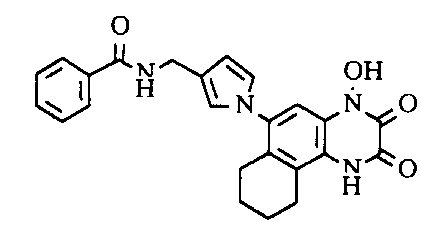
a) N- (2,5-Dimethoxytetrahydrofuran-3-ylmethyl)-benzoesäureamid
   2 g (12,4 mMol) 2,5-Dimethoxyl-3-aminomethyl-tetrahydrofuran und 3,4 ml (24,8 mMol) Triethylamin wurden in 50 ml wasserfreiem Tetrahydrofuran gelöst. Bei 0°C wurden 1,7 g (12,4 mMol) Benzoylchlorid, gelöst in 20 ml wasserfreiem Tetrahydrofuran, zugetropft. Man rührte 1 h. Der Ansatz wurde filtriert und das Filtrat im Vakuum eingeengt. Der Rückstand wurde aus Ether/Petrolether umgefällt. Man erhielt 2,4 g Produkt, das ohne weitere Reinigung verwendet wurde.
b) 9-(3-Benzoylaminomethyl-1-pyrrolyl)-1-hydroxy-5,6,7,8-tetrahydrobenzo[f]chinoxalin
   1,2 (4,8 mMol) Substanz des Beispiels 5 und 1,3 g (4,8 mMol) des Produktes 7a wurden in 70 ml Eisessig 10 min unter Rückfluß gekocht. Danach wurde der Ansatz auf Eiswasser gegossen und der anfallende Niederschlag abgesaugt. Man erhielt 1,6 g (77 %) Produkt, Schmp. 212°C (Zersetzung).
   ¹H-NMR (D₆-DMSO): δ = 1,6 (2H); 1,8 (2H); 2,5 (2H); 2,8 (2H); 4,4 (2H); 6,2 (1H): 6,8 (2H); 7,2 (1H); 7,4 - 7,6 (3H); 7,9 (2H); 8,8 (1H); 11,4 (1H) und ca. 12 (breit) ppm.

### Beispiel 8

1-Benzyloxy-9-(2,5-dimethyl-pyrrol-1-yl)-5,6,7,8-tetrahydrobenzo[f]-chinoxalin-2,3(1H,4H)-dion

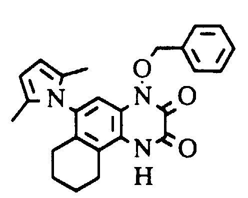

2,0 g (6,2 mMol) Substanz des Beispiels 2 und 1,1 ml (9,4 mMol) Benzylbromid wurden in 150 ml Ethanol gelöst, mit 50 ml eines Phosphat-Puffers (1,22 g Kaliumdihydrogenphosphat, 5,7 g Dinatriumhydrogenphosphat in 100 ml Wasser) versetzt und 2 h bei Raumtemperatur gerührt. Danach wurde das Ethanol im Vakuum entfernt, die resultierende wäßrige Phase mit 1 M Salzsäure angesäuert und mit Essigester extrahiert. Die organische Phase wurde getrocknet, im Vakuum eingeengt und der Rückstand mit wenig Ethanol behandelt. Man erhielt 2,1 g (83 %) Produkt, Schmp. 220°C (Zersetzung).

¹H-NMR (D₆-DMSO): δ = 1,6 (2H); 1,7 - 1,9 (8H); 2,0 (2H); 2,8 (2H); 5,2 (2H); 5,8 (2H); 7,0 (1H); 7,4 - 7,6 (5H) und ca. 11,5 (breit) ppm.

### Beispiel 9

9-(2,5-Dimethylpyrrol-1-yl)-1-ethoxycarbonylmethoxy-5,6,7,8-tetra-hydrobenzo[f]chinoxalin-2,3(1H,4H)-dion

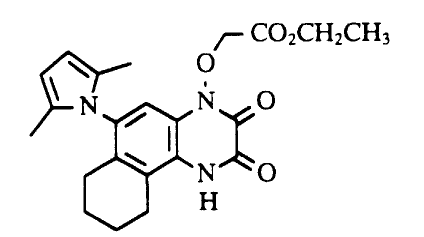

3,5 g (10,8 mMol) Substanz des Beispiels 2 und 1,8 ml (16,4 mMol) Bromessigsäureethylester wurden analog Beispiel 8 umgesetzt. Man erhielt 3,2g (73 %) Produkt, Schmp. 157 - 158°C.

¹H-NMR (D₆-DMSO): δ = 1,2 (3H); 1,6 (2H): 1,9 (2H); 1,95 (6H); 2,1 (2H); 2,9 (2H); 4,2 (2H); 5,0 (2H); 5,9 (2H); 7,4 (2H) und ca. 11,5 (1H) ppm.

### Beispiel 10

1-Carboxymethyloxy-9-(2,5-dimethylpyrrol-1-yl)-5,6,7,8-tetrahydro-benzo[f]chinoxalin-2,3 (1H,4H)-dion

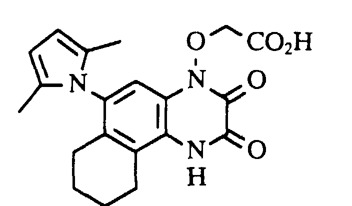

1,5 g (3,7 mMol) Substanz des Beispiels 9 wurden in 70 ml Tetrahydrofuran gelöst und bei Raumtemperatur mit 0,26 g (10,9 mMol) Lithiumhydroxid, gelöst in 10 ml Wasser, versetzt. Man rührte 2 h. Danach wurde das Tetrahydrofuran im Vakuum entfernt, die resultierende wäßrige Phase mit 1 M Salzsäure angesäuert und der anfallende Niederschlag abgesaugt. Man erhielt 1,1 g (79 %) Produkt, Schmp. > 260°C.

¹H-NMR (D₆-DMSO): δ = 1,6 (2H); 1,7 - 1,9 (8H); 2,1 (2H); 2,8 (2H); 4,8 (2H); 5,8 (2H); 7,4 (1H) und ca. 11,5 (1H) ppm.

### Beispiel 11

9-(3-Formyl-1-pyrrolyl)-5,6,7,8-tetrahydrobenzo[f]-chinoxalin-2,3-(1H,4H)-dion

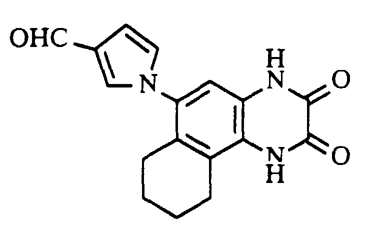

0,5 g (1,5 mMol) Substanz des Beispiels 1 wurden in 25 ml Eisessig unter Rückfluß erhitzt. Anschließend gab man 0,09 g (1,6 mMol) Eisenpulver portionsweise zu und erhitzte für weitere 10 min. Danach wurde alles auf Wasser gegeben und der Niederschlag abgesaugt. Man erhielt 0,44 g (94 %) Produkt, Schmp. > 250°C.

¹H-NMR (D₆-DMSO): δ =1,6 (2H); 1,8 (2H); 2,4 (2H); 2,8 (2H); 6,6 (1H); 6,9 (); 7,0 (1H); 7,8 (1H); 9,8 (1H); 11,2 (1H) und 12 (1H) ppm.

### Beispiel 12

9-(2,5-Dimethyl-1-pyrrolyl)-5,6,7,8-tetrahydrobenzo[f]chinoxalin-2,3(1H,4H)-dion 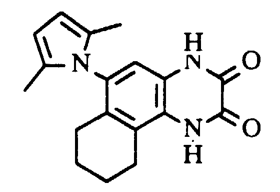
a) 9-Amino-5,6,7,8-tetrahydrobenzo[f]chinoxalin-2,3(1H,4H)-dion
   20 g (0,08 Mol) des Produktes 1c wurden in 500 ml Eisessig unter Rückfluß erhitzt und portionsweise mit 14 g (0,25 Mol) Eisenpulver versetzt. Anschließend wurde eine weitere Stunde unter Rückfluß erhitzt. Der Ansatz wurde danach heiß filtriert und das Filtrat im Vakuum eingeengt. Der anfallende Rückstand wurde mit heißem Wasser behandelt und das Produkt abgesaugt. Man erhielt 17 g (61 %).
   ¹H-NMR (CD₃COOD): δ = 1,8 (2H); 1,9 (2H); 2,5 (2H), 2,7 (2H) und 6,6 (1H) ppm.
b) 9- (2,5-Dimethyl-1-pyrrolyl)-5,6,7,8-tetrahydrobenzo[f]chinoxalin-2,3(1H,4H)-dion
   3 g (11,5 mMol) des Produktes 12a und 1,3 g (11,5 mMol) Hexan-2,5-dion wurden in 150 ml 30 min unter Rückfluß gekocht. Anschließend wurde der Ansatz im Vakuum eingeengt und der anfallende Rückstand mit Wasser behandelt und abgesaugt. Das Rohprodukt wurde mit wenig Essigester ausgekocht. Man erhielt 1,8 g (49 %) Produkt, Schmp. > 265°C.
   ¹H-NMR (D₆-DMSO): δ = 1,6 (2H), 1,7 - 1,9 (8H); 2,0 (2H); 2,8 (2H); 5,8 (2H); 6,8 (1H); 11,2 (1H) und 12 (1H) ppm.

### Beispiel 13

9-(1-Pyrrolyl)-5,6,7,8-tetrahydrobenzo[f]chinoxalin-2,3-(1H,4H)dion

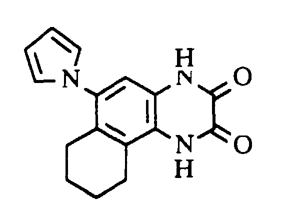

1 g (3,8 mMol) des Produktes 12a und 0,5 g (3,8 mMol) 2,5-Dimethoxytetrahydrofuran wurden analog Vorschrift 1d umgesetzt. Man erhielt 0,8 g (67 %) des Produktes. Schmp. > 250°C.

¹H-NMR (D₆-DMSO): δ = 1,6 (2H); 1,8 (2H); 2,4 (2H); 2,7 (2H); 6,2 (2H); 6,9 (3H); 11,2 (1H) und 12 (1H) ppm.

### Beispiel 14

9-(3-(4-Benzylpiperazin-1-yl)methylpyrrol-1-yl)-5,6,7,8-tetrahydro-benzo[f]chinoxalin-2,3(1H,4H)-dion-difumarat

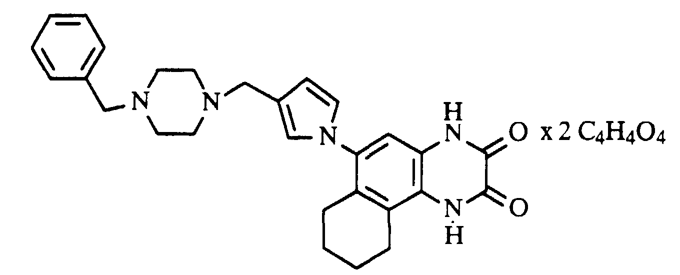

1,3 g (4,2 mMol) Substanz des Beispiels 11, 1,5 g (8,4 mMol) 4-Benzylpiperazin und 0,25 g (4,2 mMol) Essigsäure wurden in 100 ml Dimethylformamid gelöst und bei Raumtemperatur portionsweise mit 0,26 g (4,2mMol) Natriumcyanoborhydrid versetzt. Es wurde 16 h gerührt. Anschließend wurde der Ansatz im Vakuum eingeengt und der Rückstand in wäßriger Natriumhydrogenkarbonat-Lösung aufgeschlämmt. Der Niederschlag wurde abgesaugt, in Tetrahydrofuran gelöst, mit 2fach äquimolarer Menge Fumarsäure in Ethanol versetzt und aufgekocht. Nach dem Abkühlen wurde das Produkt abgesaugt. Man erhielt 1,2 g (50 %) Produkt, Schmp. > 250°C.

¹H-NMR (D₆-DMSO): δ = 1,6 (2H); 1,8 (2H); 2,3 - 2,8 (12H); 3,5 (2h); 3,6 (2H); 6,2 (1H); 6,4 (4H); 6,8 (2H); 6,9 (1H); 7,2 - 7,4 (5H); 11,1 (1H) und 11,9 (1H) ppm.

### Beispiel 15

9-(3-Trifluormethylamidomethyl-1-pyrrolyl)-5,6,7,8-tetrahydrobenzo-[f]chinoxalin-2,3(1H,4H)-dion

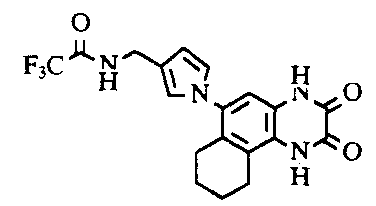

1,1 g (4,8 mMol) Substanz des Produktes 12a und 1,2 g (4,8 mMol) des Produktes 4a wurden analog Vorschrift 1e umgesetzt. Man erhielt 1,4g (76 %) des Produktes. Schmp. > 240°C.

¹H-NMR (D₆-DMSO): δ = 1,6 12H; 1,8 (2H); 2,4 (2H); 2,8 (2H); 4,2 (2H); 6,2 (1H); 6,7 (2H); 6,8 (1H); 9,8 (1H); 11,2 (1H) und 12,0 (1H) ppm.

### Beispiel 16

9-(3-Aminomethyl-1-pyrrolyl)-5,6,7,8-tetrahydrobenzo[f]chinoxalin-2,3(1H,4H)-dion

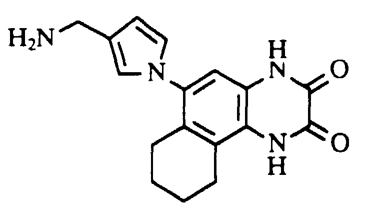

1,3 g (3,3 mMol) des Produktes 15 wurden in 50 ml Tetrahydrofuran gelöst, mit 0,32 g (13,3 mMol) Lithiumhydroxid, gelöst in 50 ml Wasser, versetzt. Man rühre 1 h bei Raumtemperatur. Anschließend wurde das Tetrahydrofuran im Vakuum entfernt und die resultierende wäßrige Phase mit 1 M Salzsäure neutralisiert. Der anfallende Niederschlag wurde abgesaugt. Man erhielt 1,1 g (100 %) Produkt, Schmp.: > 220°C.

¹H-NMR (CD₃COOD): δ = 1,75 (2H); 1,9 (2H); 2,6 (2H); 2,9 (2H); 4,2 (2H); 6,4 (1H); 6,8 (1H); 7,0 (1H) und 7,1 (1H) ppm.

### Beispiel 17

N-(1-(5,6,7,8-Tetrahydrobenzo[f]chinoxalin-2,3(1H,4H)-dion-9-yl)pyrrol-3-yl)methyl-N'-phenylharnstoff

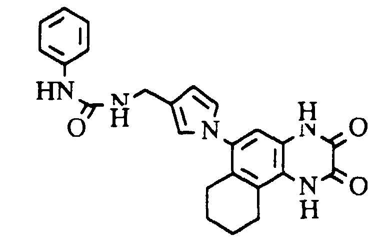

1,0 g (3,4 mMol) Substanz des Beispiels 16 und 0,44 g (3,7 mMol) Phenylisocyanat wurden in 30 ml wasserfreiem Dimethylformamid 15 min auf 110°C erhitzt. Nach dem Abkühlen wurde der angefallene Niederschlag abgesaugt und mit Ethanol gewaschen. Man erhielt 1,12 g (66 %) Produkt, Schmp. > 240°C.

¹H-NMR (D₆-DMSO): δ = 1,6 (2H); 1,8 (2H); 2,4 (2H); 2,8 (2H); 4,2 (2H); 6,2 (1H); 6,3 (1H); 6,8 (1H); 6,9 (3H); 7,2 (2H); 7,4 (2H); 8,4 (1H); 11,2 (breit) und 11,9 (breit) ppm.

### Beispiel 18

9-(3-(4-(1,1-Diphenylmethyl)piperazin-1-yl)methyl-pyrrol-1-yl)-1-hydroxy-5,6,7,8-tetrahydrobenzo[f]chinoxalin-2,3(1H,4H)-dion

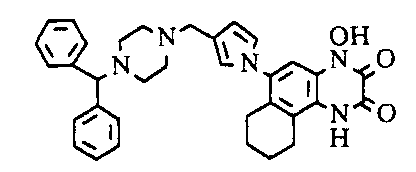

1,6 g (4,9 mMol) des Beispiels 1 und 2,5 g (9,8 mMol) 4-(1,1-Diphenylmethyl)piperazin wurden analog Beispiel 14 umgesetzt. Man erhielt 2,4 g (84 %) Produkt, Schmp. > 200°C.

¹H-NMR (CD₃COOD) : δ = 1,7 (2H); 1,9 (2H); 2,5 (2H); 2,9 (2H); 3,0 -3,7 (8H); 4,3 (2H); 4,8 (1H); 6,4 (1H); 6,9 (1H); 7,1 (1H) und 7,2 -7,6 (11H) ppm.

### Beispiel 19

9-(3-(4-Benzylpiperazin-1-yl)methylpyrrol-1-yl)-1-hydroxy-5,6,7,8-tetrahydrobenzo[f]chinoxalin-2,3(1H,4H)-dion

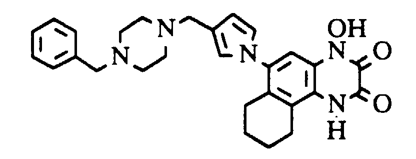

1,5 g (4,6 mMol) 1 und 1,6 g (9,2 mMol) 4-Benzylpiperazin wurden analog Vorschrift 14 umgesetzt. Man erhielt 1,1 g (50 %) Produkt, Schmp. > 200°C.

¹H-NMR (CD₃COOD): δ = 1,7 (2H); 1,9 (2H); 2,5 (2H); 2,9 (2H); 3,6 -3,8 (8H); 4,3 (2H); 4,4 (2H); 6,4 (1H); 6,9 (1H); 7,05 (1H) und 7,4 - 7,6 (6H) ppm.

### Beispiel 20

1-Ethoxycarbonylmethyl-9-(3-formyl-pyrrol-1-yl)-5,6,7,8-tetrahydro-benzo[f]chinoxalin-2,3(1H,4H)-dion

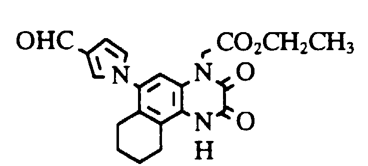

7 g (22,6 mMol) Substanz des Beispiels 11 wurden unter Stickstoff in 100 ml wasserfreiem Dimethylformamid gelöst und bei Raumtemperatur portionsweise mit 0,7 g (22,6 mMol) Natriumhydrid (80 %ig) versetzt. Nach 1 h tropfte man 2,8 ml (24,9 mMol) Bromessigsäureethylester zu und rührte 16 h. Anschließend wurde der Ansatz auf Wasser gegeben und mit Essigester extrahiert. Die organische Phase wurde getrocknet und im Vakuum eingeengt. Der Rückstand wurde chromatographisch an Kieselgel gereinigt. Man erhielt 4,5 g (51 %) Produkt, Schmp. > 250°C.

¹H-NMR (D₆-DMSO): δ = 1,2 (3H); 1,6 (2H); 1,8 (2H); 2,4 (2H); 2,8 (2H); 4,1 (2H); 5,0 (2H); 6,6 (1H); 7,0 (1H); 7,3 (1H); 7,8 (1H); 9,8 (1H) und 11,4 (1H) ppm.

### Beispiel 21

9-(3-(4-(1,1-Diphenylmethyl)piperazin-1-yl)methyl-pyrrol-1-yl)-1-ethoxycarbonylmethyl-5,6,7,8-tetrahydrobenzo[f]chinoxalin-2,3-(1H,4H)-dion

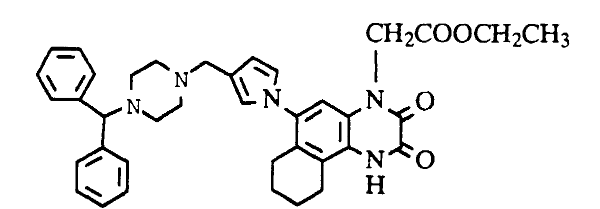

1,0 g (2,5 mMol) Substanz des Beispiels 20 und 1,3 g (5,1 mMol) 4-(1,1-Diphenylmethyl)piperazin wurden analog Beispiel 14 umgesetzt. Man erhielt 0,9 g (57 %) Produkt, Schmp. > 200°C.

¹H-NMR (D₆-DMSO): δ = 1,2 (3H); 1,6 (2H); 1,8 (2H); 2,4 (2H); 2,8 (2H); 2,9 - 3,6 (10H); 4,2 (3H); 5,0 (2H); 6,4 (1H); 6,9 (1H); 7,0 (1H); 7,2 (1H); 7,2 - 7,6 (10H) und 11,3 (1H) ppm.

### Beispiel 22

9-(3-(4-Benzylpiperazin-1-yl)methylpyrrol-1-yl)-1-ethoxycarbonylmethyl-5,6,7,8-tetrahydrobenzo[f]-chinoxalin-2,3(1H,4H)-dion

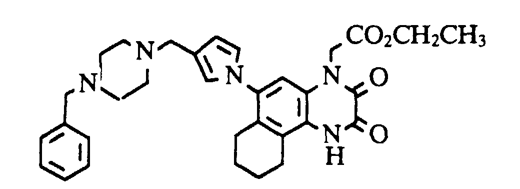

1 g (2,5 mMol) Substanz des Beispiels 21 und 0,9 g (5,1 mMol) 4-Benzylpiperazin wurden analog Beispiel 14 umgesetzt. Man erhielt 0,8 (58%) Produkt, Schmp. > 220°C.

¹H-NMR (D₆-DMSO): δ = 1,2 (3H); 1,6 82H); 1,8 (2H); 2,4 - 2,7 (6H); 2,8 (2H); 3,2 - 3,6 (8H); 4,2 (2H); 5,0 (2H); 6,2 (1H); 6,6 (2H, Fumarat); 6,8 (2H); 7,1 (1H); 7,2 - 7,4 (5H) und ca. 11,3 (1H) ppm.

### Beispiel 23

1-Carboxymethyl-9-(3-formyl-pyrrol-1-yl)-5,6,7,8-tetrahydrobenzo-[f]-chinoxalin-2,3(1H, 4H)-dion

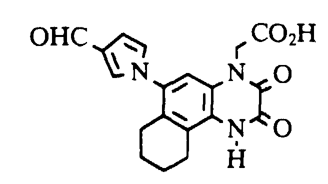

1 g (2,5 mMol) Substanz des Beispiels 20 wurden in 3 ml Tetrahydrofuran gegeben und mit 10 ml (5 mMol) einer 0,5 molaren Lithiumhydroxid-Lösung versetzt. Es wurde 1 h bei Raumtemperatur gerührt. Anschließend wurde das Tetrahydrofuran im Vakuum entfernt und die resultierende wäßrige Phase mit verdünnter Salzsäure neutralisiert. Der ausgefallene Niederschlag wurde abgesaugt. Man erhielt 0,7 g (76%) Produkt, Schmp. > 250°C.

¹H-NMR (D₆-DMSO): δ = 1,6 (2H); 1,8 (2H); 2,4 (2H); 2,8 (2H); 4,8 (2H); 6,6 (1H); 7,0 (1H); 7,2 (1H9; 7,7 (1H), 9,7 (1H) und ca. 11,3 (breit) ppm.

### Beispiel 24

1-Carboxymethyl-9-(3-(4-(1,1-diphenylmethyl)-piperazin-1-yl)methyl-pyrrol-1-yl)-5,6,7,8-tetrahydrobenzo[f]chinoxalin-2,3(1H,4H)-dion

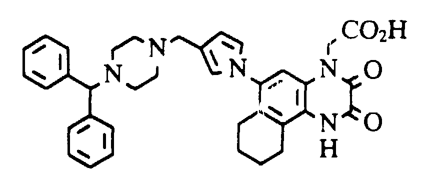

0,8 g (1,3 mMol) Substanz des Beispiels 21 wurden analog Beispiel 23 hydrolysiert. Man erhielt 0,76 g (85 %) Produkt, Schmp. > 220°C.

¹H-NMR (CD₃COOD): δ = 1,7 (2H); 1,9 (2H); 2,5 (2H); 2,9 (2H); 3,6 (4H); ca. 3,8 (breit, 4H); 4,3 (2H); 5,1 (2H); 5,4 (1H); 6,4 (1H); 6,8 (1H); 7,1 (1H); 7,3 (1H); 7,3 - 7,5 (6H) und 7,8 (4H) ppm.

### Beispiel 25

9-(2-Acetamidomethyl-1-pyrrolyl)-1-ethoxycarbonylmethyl-5,6,7,8-tetrahydrobenzo[f]chinoxalin-2,3-(1H,4H)-dion 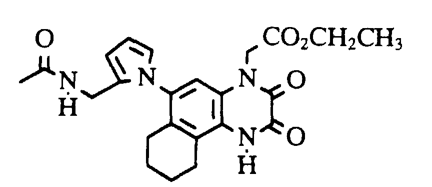
a) N-Benzyl-N-(2,4-dinitro-5, 6,7, 8-tetrahydronaphthyl-1-)-oxalsäuremonoethylesteramid
   109,5 g (0,32 Mol) des Produktes 1b wurden in 1,25 L wasserfreiem Tetrahydrofuran gelöst und bei Raumtemperatur portionsweise mit 10,7 g (0,36 Mol) 80 %igem Natriumhydrid versetzt. Anschließend wurde eine Lösung aus 55,4 g = 38,4 ml (0,32 Mol) Benzylbromid in 100 ml wasserfreiem Tetrahydrofuran zugetropft. Nachdem man noch 50 ml wasserfreies Dimethylformamid zugegeben hatte, wurde alles 7 h unter Rückfluß gekocht. Anschließend wurde der Ansatz im Vakuum eingeengt, der Rückstand zwischen Wasser und Essigester verteilt, die organische Phase getrocknet und im Vakuum eingeengt. Dieser Rückstand wurde an Kieselgel chromatographisch (Fließmittel: Cyclohexan/Essigester = 1/1) gereinigt. Man erhielt 108,4 g (78 %) Produkt.
   ¹H-NMR (D₆-DMSO): δ = 1,0 (3H); 1,4 - 1,9 (4H); 2,5 - 3,0 (4H); 4,0 (2H); 4,6 (1H); 5,1 (1H); 7,0 - 7,4 (5H) und 8,4 (1H) ppm.
b) 9-Amino-4-benzyl-5,6,7,8-tetrahydrobenzo[f]chinoxalin-2,3-(1H,4H)-dion
   107 g (0,25 Mol) des Produktes 25a wurden in 1,5 L Eisessig unter Rückfluß gekocht. Danach wurden portionsweise 77 g (1,4Mol) Eisenpulver zugegeben und alles 30 min unter Rückfluß gekocht. Anschließend wurde der Ansatz auf viel Wasser gegossen und der anfallende Niederschlag abgesaugt. Man erhielt 42,4 g (53 %) Produkt, Schmp. 290°C.
   ¹H-NMR (D₆-DMSO): δ = 1,4 (2H); 1,6 (2H); 2,4 (2H); 2,6 (2H); 4,9 (2H); 5,2 (2H); 6,4 (1H); 76,0 - 7,4 (5H) und 11,6 (1H) ppm.
c) 9-Amino-4-benzyl-1-ethoxycarbonylmethyl-5,6,7,8-tetrahydrobenzo[f]chinoxalin-2,3-(1H,4H)-dion
   41,5 g (0,13 Mol) des Produktes 25b wurden in 700 ml wasserfreiem Dimethylformamid gelöst und bei Raumtemperatur portionsweise mit 4,3 g (0,14 Mol) 80 %igem Natriumhydrid versetzt. Anschließend tropfte man eine Lösung aus 21,7 g (0,13 Mol) Bromessigsäureethylester in 100 ml wasserfreiem Dimethylformamid gelöst zu. Nach 1 h Rühren bei Raumtemperatur wurde der Ansatz auf Wasser gegeben und mit Natriumhydrogenkarbonat schwach alkalisch gestellt. Durch Zugabe von Natriumchlorid wurde das Produkt ausgefällt, abgesaugt und gut mit Ether gewaschen wurde. Man erhielt 45,4 g (86 %), Schmp. 231°C.
   ¹H-NMR (D₆-DMSO): δ = 1,3 (3H);1,5 (2H), 1,8 (2H), 2,4 (2H); 2,8 (2H); 4,2 (2H); 4,8 (1H); 5,0 (1H); 5,3 (2H); 6,4 (1H) und 7,0 - 7,4 (5H) ppm.
d) 9-Amino-1-ethoxycarbonylmethyl-5,6,7,8-tetrahydrobenzo[f]chinoxalin-2,3(1H,4H)-dion
   44,5 g (0,11 Mol) des Produktes 25c wurden 1000 ml Dimethylformamid gelöst und nach Zugabe von 50 ml Essigsäure und 5 g Palladium/Kohle (10 %ig) hydriert. Anschließend wurde filtriert und das Filtrat im Vakuum eingeengt. Der Rückstand wurde aus Ethanol/Ether umkristallisiert. Man erhielt 30,4 g (88 %) Produkt, Schmp. 238°C.
   ¹H-NMR (D₆-DMSO): δ = 1,3 (3H); 1,8 (4H); 2,4 (2H); 2,7 (2H); 4,2 (2H); 4,9 (2H); 4,9 (2H), 6,3 (1H) und ca. 11 (1H) ppm.
e) N-[(2,5-Dimethoxy-2-tetrahydrofuranyl)methyl]-essigsäureamid
   11,5 (71,3 mMol) des 2-Aminomethyl-2,5-dimethoxytetrahydrofuran und 20 ml (143 mMol) Triethylamin wurden in 150 ml wasserfreiem Tetrahydrofuran gelöst. Bei 0°C wurde eine Lösung von 5 ml (71,3 mMol) Acetylchlorid in 50 ml Tetrahydrofuran zugetropft und 1 h gerührt. Der Niederschlag wurde abfiltriert und das Filtrat im Vakuum eingeengt. Man erhielt 12 g Rohprodukt, das ungereinigt weiter umgesetzt wurde.
f) 9-(2-Acetamidomethyl-1-pyrrolyl)-1-ethoxycarbonylmethyl-5,6,7,8-tetrahydrobenzo[f]chinoxalin-2,3(1H,4H)-dion
   3,2 g (10 mMol) des Produktes 25e und 2,5 g (812,5 mMol) des Produktes 25f wurden in 50 ml Eisessig 20 min unter Rückfluß gekocht. Danach wurde alles auf Eiswasser gegossen. Der Niederschlag wurde abgesaugt und das Filtrat mit Essigester extrahiert. Die organische Phase wurde getrocknet und im Vakuum eingeengt. Der Rückstand wurde mit dem obigen Niederschlag vereinigt. Man erhielt 1,9 g (43 %) Produkt, Schmp. 269°C.
   ¹H-NMR (D₆-DMSO): δ = 1,2 (3H); 1,5 - 1,9 (7H); 2,0 - 2,4 (2H); 2,8 (2H); 3,8 (1H); 4,0 (1H); 4,2 (2H); 5,0 (2H); 6,2 (2H); 6,7 (1H): 7,3 (1H); 9,1 (1H) und ca. 11,4 (1H) ppm.

### Beispiel 26

1-Ethoxycarbonylmethyl-9-(3-trifluoracetamidomethyl-1-pyrrolyl)-5,6,7,8-tetrahydrobenzo[f]chinoxalin-2,3-(1H,4H)-dion 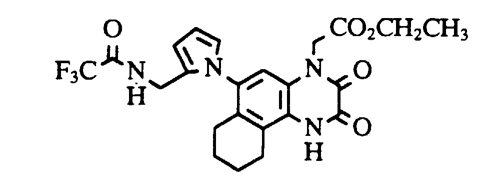
a) N- (5,6,7,8-Tetrahydro-1-napthyl)-essigsäureamid
   Zu 100 ml (0,72 Mol) 5,6,7,8-Tetrahydronaphtyl-1-amin, gelöst in 800 ml Tetrahydrofuran, wurden bei Raumtemperatur 100 ml (1,1 Mol) Essigsäureanhydrid getropft. Anschließend wurde 1 h bei 40°C gerührt. Nach dem Abkühlen wurde des Produkt durch Zugabe von Petrolether ausgefällt. Man erhielt 120 g (94 %) Produkt.
   ¹-H-NMR (D₆-DMSO): δ = 1,75 (4H); 2,1 (3H); 2,6 (2H); 2,8 (2H); 6,9 (1H); 7,1 (1H); 7,2 (1H) und 9,1 (1H) ppm.
b) N-(2,4-Dinitro-5,6,7,8-tetrahydro-1-naphthyl)-essigsäureamid
   120 g (0,63 Mol) des Produktes 26a wurden in 1200 ml konzentrierter Schwefelsäure gelöst. Bei 10°C wurden innerhalb von 2h 80 ml 98 %ige Salpetersäure zugetropft. Danach wurde noch 30 min gerührt und anschließend alles auf Eis gegossen. Der ausgefallene Niederschlag wurde abgesaugt und aus Ethanol umkristallisiert. Ausbeute: 86 g (49 %); Schmp. 203°C.
   ¹H-NMR (D₆-DMSO): δ = 1,8 (4H); 2,1 (3H); 2,8 (2H); 3,0 (2H); 8,4 (1H) und 10,1 (1H) ppm.
c) 2,4-Dinitro-5,6,7,8-tetrahydro-1-naphthylamin
   80 g (0,29 Mol) des Produktes 26b wurden in einem Gemisch aus 250 ml Ethanol, 250 ml konzentrierter Salzsäure und 100 ml Wasser 3 h unter Rückfluß gekocht. Der ausgefallene Niederschlag wurde anschließend abgesaugt. Man erhielt 55 g (85 %) Produkt, Schmp. 175 - 176°C.
   ¹H-NMR (D₆-DMSO): δ = 1,7 (2H); 1,9 (2H); 2,5 (2H); 3,0 (2H); 7,9 (2H, NH₂) und 8,6 (1H) ppm.
d) 2-Amino-4-nitro-5,6,7,8-tetrahydro-1-napthylamin
   123 g (3,1 Mol) Natronlauge und 123 g (0,48 Mol) Schwefel wurden 1 L Wasser bis zum Aufklaren der Lösung (ca. 1 h) auf 100°C erwärmt. Danach wurden 1 L Methanol und portionsweise 54 g (0,23 Mol) des Produktes 26c zugegeben. Es wurde anschließend noch 30 min gerührt. Das Methanol wurde im Vakuum entfernt und die wäßrige Phase mit Eis gekühlt. Der anfallende Niederschlag wurde abgesaugt. Man erhielt 34 g (73 %) Produkt, Schmp. 181 - 182°C.
   ¹H-NMR (D₆-DMSo): δ = 1,7 (2H); 1,8 (2H); 2,4 (2H); 2,9 (2H); 4,9 (2H); 5,6 (2H) und 7,3 (1H) ppm.
e) 9-Nitro-5,6,7,8-tetrahydrobenzo[f]chinoxalin-2,3(1H,4H)-dion
   33,5 g (0,16 Mol) des Produktes 26d wurden in 300 ml Oxalsäurediethylester 3 h unter Rückfluß gekocht. Anschließend wurde der Niederschlag abgesaugt. Man erhielt 29,7 g (71 %) Produkt, Schmp. > 250°C.
   ¹H-NMR (D₆-DMSO): δ = 1,6 - 1,9 (4H); 2,8 (2H); 2,9 (2H); 7,6 (1H); 11,4 (1H) und ca. 12 (breit) ppm.
f) 1-Ethoxycarbonylmethyl-9-nitro-5,6,7,8-tetrahydrobenzo[f]-chinoxalin-2,3(1H,4H)-dion
   29 g (0,11 Mol) des Produktes 26e wurden unter Schutzgas in 300ml Dimethylformamid gelöst und bei Raumtemperatur mit 3,3 g (0,11 Mol) Natriumhydrid (80 %ig) portionsweise versetzt. Man rührte anschließend 1 h. Danach wurden 12,9 ml (0,12 Mol) Bromessigsäureethylester zügig zugetropft und 2 h bei Raumtemperatur gerührt. Anschließend wurden 50 ml Essigsäure zugetropft und der Reaktionsansatz im Vakuum eingeengt. Der Rückstand wurde chromatographisch (Fließmittel = Toluol: Aceton: Eisessig = 40:20:1) gereinigt. Man erhielt 12,5 g (33 %) Produkt, Schmp. 217 - 219°C.
   ¹H-NMR (D₆-DMSO): δ = 1,2 (3H); 1,7 (2H); 1,8 (2H); 2,8 (2H); 2,9 (2H); 4,2 (2H); 5,0 (2H); 7,8 (1H) und 11,5 (1H) ppm.
g) 9-Amino-1-ethoxycarbonylmethyl-5,6,7,8-tetrahydrobenzo[f]chinoxalin-2,3(1H, 4H)-dion
   5 g (14,4 mMol) des Produktes 26f wurden in 150 ml Dimethylformamid gelöst und nach Zugabe von 0,5 g Palladium/Kohle (10%) hydriert. Anschließend wurde filtriert und das Filtrat im Vakuum eingeengt. Man erhielt 3,9 g (87 %) Produkt, Schmp. > 250°C.
   ¹H-NMR (D₆-DMSO): δ = 1,2 (3H); 1,8 (4H); 2,4 (2H); 2,7 (2H); 4,2 (2H); 4,9 (4H); 6,4 (1H) und 10,9 (1H) ppm.
h) 1-Ethoxycarbonylmethyl-9-(3-trifluoracetamidomethyl-1-pyrrolyl)-5,6,7,8-tetrahydro-benzo[f]chinoxalin-2,3(1H,4H)-dion
   3,5 g (11,0 mMol) des Produktes 26g und 3,5 g (13,8 mMol) des Produktes 4a wurden in 100 ml konzentrierter Essigsäure 10 min unter Rückfluß gekocht. Anschließend wurde der Ansatz im Vakuum eingeengt und der Rückstand mit wenig Ethanol behandelt. Der Niederschlag wurde abgesaugt. Man erhielt 4,4 g (82 %) Produkt, Schmp. 242 - 243°C.
   ¹H-NMR (D₆-DMSO): δ = 1,2 (3H); 1,6 (2H); 1,8 (2H), 2,4 (2H); 2,8 (2H); 4,2 (2H); 4,3 (2H); 5,0 (2H); 6,2 (1H); 6,8 (2H); 7,1 (1H) 9,8 (1H) und 11,4 (1H) ppm.

### Beispiel 27

9-(3-Aminomethyl-1-pyrrolyl)-1-carboxymethyl-5,6,7,8-tetrahydrobenzo[f]chinoxalin-2,3(1H,4H)-dion

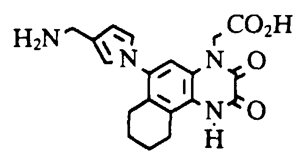

4,3 g (8,7 mMol) des Beispiels 26 wurden in 20 ml Tetrahydrofuran suspendiert und mit 0,84 g (35,1 mMol) Lithiumhydroxid, gelöst in 50ml Wasser, versetzt. Es wurde 1 h bei Raumtemperatur gerührt, anschließend das Tetrahydrofuran im Vakuum entfernt und die resultierende wäßrige Phase mit 1 M Salzsäure neutralisiert. Der ausgefallene Niederschlag wurde abgesaugt. Man erhielt 2,3 g (72%) Produkt, Schmp. > 250°C.

¹H-NMR (CD₃COOD): δ =1,7 (2H), 1,9 (2H), 2,5 (2H); 2,9 (2H); 4,2 (2H); 5,1 (2H); 6,4 (1H); 6,8 (1H); 7,0 (1H); und 7,1 (1H) ppm.

### Beispiel 28

N-(1-(1-Carboxymethyl-5,6,7,8-tetrahydro-benzo[f]chinoxalin-2,3-(1H, 4H)-dion-9-yl)-pyrrol-3-yl)methyl-N'-phenyl-harnstoff

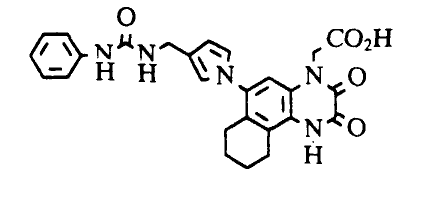

0,7 g (1,9 mMol) Substanz des Beispiels 27 und 0,24 g (2,0 mMol) Phenylisocyanat wurde in 5 ml wasserfreiem Dimethylformamid 15 min auf 110°C erwärmt. Nach dem Abkühlen fällte man durch Zugabe von Ether das Produkt aus. Man erhielt 0,86 g (97 %) Produkt, Schmp. 198°C (Zersetzung).

¹H-NMR (D₆-DMSO): δ = 1,7 (2H) ; 1,8 (2H) ; 2,5 (2H); 2,8 (2H); 4,2 (2H); 5,0 (2H); 6,2 (1H); 6,3 (1H); 6,8 (2H); 6,9 (1H); 7,1 (1H); 7,3 (2H); 7,4 (2H); 8,5 (1H); 11,4 (1H) und ca. 13,5 (breit) ppm.

### Beispiel 29

N-(1-(1-Carboxymethyl-5,6,7,8-tetrahydrobenzo[f]chinoxalin-2,3-(1H, 4H)-dion-9-yl)-pyrrol-3-ylmethyl-N'-(4-nitrophenyl)-harnstoff

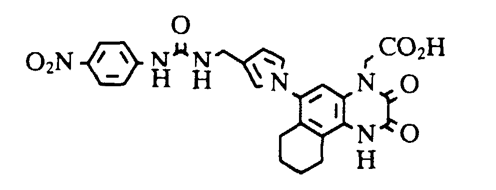

0,4 g (2,2 mMol) Substanz des Beispiels 27 und 0,38 g (2,3 mMol) 4-Nitrophenylisocyanat wurden in 5 ml wasserfreiem Dimethylformamid 10 min auf 120°C erwärmt. Danach fügte man nochmals 0,38 g 4-Nitrophenylisocyanat zu. Nach weiteren 5 min wurde abgekühlt und filtriert. Das Filtrat wurde mit Methylenchlorid versetzt, wobei das Produkt ausfiel. Man erhielt 0,75 g (66 %), Schmp. > 180°C.

¹H-NMR (D₆-DMSO): δ = 1,6 (2H); 1,8 (2H); 2,4 (2H); 2,9 (2H); 4,2 (2H); 4,9 (2H); 6,2 (1H); 6,8 (3H); 7,1 (1H); 7,6 (2H); 8,2 (2H); 9,3 (1H); 11,3 (1H) und ca. 13 (breit) ppm.

### Beispiel 30

N-(1-(1-Hydroxy-5,6,7,8-tetrahydrobenzo[f]chinoxalin-2,3-(1H,4H)-dion-9-yl)-pyrrol-3-yl)-methyl-N'-(4-nitrophenyl)-harnstoff 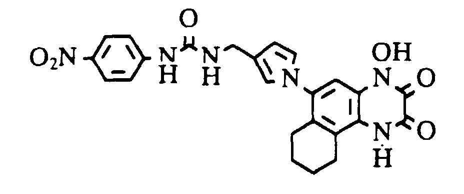
a) N-(2,5-Dimethoxy-tetrahydrofuran-3-yl)methyl-N'-(4-nitrophenyl)-harnstoff
   Zu einer Lösung aus 27 g (0,18 Mol) 3-Aminomethyl-2,5-dimethoxy-tetrahydrofuran in 150 ml Methylenchlorid tropfte man bei 0 - 5°C 25 g (0,15 Mol) 4-Nitrophenylisocyanat. Nach Erwärmen auf Raumtemperatur saugte man den anfallenden Niederschlag ab. Man erhielt 45 g eines Rohpoduktes, das ungereinigt eingesetzt wurde.
b) N-(1-(1-Hydroxy-5,6,7,8-tetrahydrobenzo[f]chinoxalin-2,3-(1H,4H)-dion-9-yl)-pyrrol-3-yl)-methyl-N'-(4-nitrophenyl)harnstoff
   2,0 g (8 mMol) des Produktes 1c und 3,3 g (10 mMol) des Produktes 30a wurden analog der Vorschrift 1d umgesetzt. Man erhielt 1,3 g (33 %) Produkt, Schmp. > 230°C.
   ¹H-NMR (D₆-DMSO): δ = 1,5 - 1,9 (4H); 2,4 (2H); 2,8 (2H); 4,2 (2H); 6,2 (1H); 6,7 (1H); 6,8 (2H); 7,1 (1H); 7,7 (2H); 8,2 (2H); 9,4 (1H) und ca. 11,5 (breit) ppm.

### Beispiel 31

N'-(4-Nitrophenyl)-N-(1-(5,6,7,8-tetrahydrobenzo[f]chinoxalin-2,3-(1H,4H)-dion-9-yl)-pyrrol-3-yl)methyl)-harnstoff

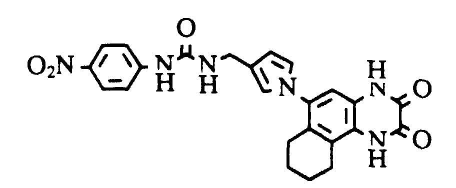

9,3 g (40 mMol) des Produktes 12a und 13 g (40 mMol) des Produktes 30a wurden analog Vorschrift 1d umgesetzt. Ausbeute: 17,1 g (90 %), Schmp.: 239°C.

¹H-NMR (D₆-DMSO): δ = 1,6 - 1,9 (4H); 2,5 - 3,0 (4H); 4,2 (2H); 6,2 (1H); 6,6 (1H); 6,7 (2H); 7,3 - 8,3 (5H); 9,2 (1H); 11,1 (1H) und 12 (breit) ppm.

### Beispiel 32

N-(1-(1-Ethoxycarbonylmethyl-5,6,7,8-tetrahydrobenzo[f]-chinoxalin-2,3-(1H,4H)-dion-9-yl)-pyrrol-3-yl)methyl-N'-(4-nitrophenyl)-harnstoff

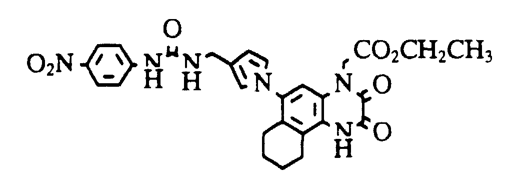

16,6 g (35 mMol) des Produktes 31 und 5,9 g (35 mMol) Bromessigsäureethylester wurden analog Beispiel 20 bei Raumtemperatur umgesetzt. Das Produkt wurde zusätzlich chromatographisch an Kieselgel (Fließmittel: Toluol/Aceton/Essigsäure = 20/10/1) gereinigt. Ausbeute: 4 g (21 %); Schmp. 246°C.

¹H-NMR (D₆-DMSO9: δ = 1,2 (3H); 1,6 - 2,0 (4H); 2,4 (2H); 2,8 (2H); 4,2 (2H); 5,0 (2H); 6,2 (1H); 6,7 (1H); 6,8 (2H); 7,2 (1H); 7,5 -8,3 (4H); 9,2 (1H) und ca. 11,3 (breit) ppm.

### Beispiel 33

9-(2-Acetamidomethyl-1-pyrrolyl)-1-hydroxy-5,6,7,8-tetrahydrobenzo-[f]chinoxalin-2,3-(1H,4H)-dion

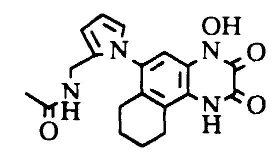

4,4 g (18 mMol) des Produktes 1c und 4,5 g (22 mMol) des Produktes 25e wurde analog Vorschrift 1d umgesetzt. Ausbeute: 2,9 g (44 %), Schmp. 295°C (Zersetzung).

¹H-NMR (D₆-DMSO): δ = 1,6 - 1,9 (7H); 2,2 (2H); 2,9 (2H); 3,9 (2H); 6,1 (2H); 6,7 (1H); 7,2 (1H); 8,0 (1H) und ca. 11,5 (breit) ppm.

### Beispiel 34

1-Hydroxy-9-(3-(4-(4-nitrophenyl)-piperazin-1-yl)-methyl-1-pyrro-lyl)-5,6,7,8-tetrahydrobenzo[f]chinoxalin-2,3-(1H,4H)-dion

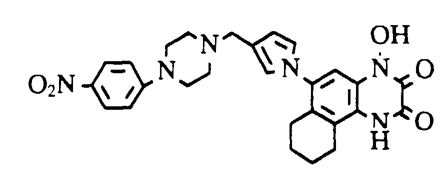

2 g (6 mMol) des Produktes 1 und 2,5 g (12 mMol) 4-(4-Nitrophenyl)-piperazin wurden analog Vorschrift 14 umgesetzt. Ausbeute: 2,7 g (86%), Schmp. > 230°C.

¹H-NMR (D₆-DMSO): δ = 1,6 (2H); 1,8 (2H); 2,4 (2H); 2,8 (2H); 3,3 -3,7 (9H); 6,1 (1H); 6,7 (1H); 6,8 (1H); 7,0 (2H); 7,2 (1H); 8,0 (2H) und ca. 11,5 (breit) ppm.

### Beispiel 35

N-(1-(1-Carboxymethyl-5,6,7,8-tetrahydrobenzo[f] chinoxalin-2,3-(1H,4H)-dion-9-yl)-pyrrol-3-yl)methyl-N'-phenyl-guanidin

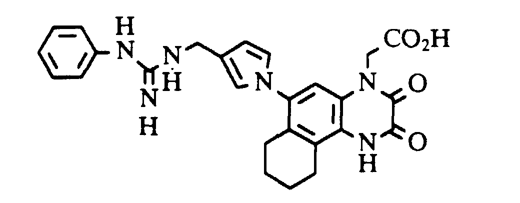

1,75 g (4,8 mMol) des Produktes 27, 1,4 g (4,8 mMol) S-Methyl-N-phenyl-isothioharnstoffhydrojodid und eine Spatelspitze 4-(N,N-Dimethylamino)-pyridin wurden in 50 ml Pyridin 6 h unter Rückfluß gekocht. Danach wurde der Ansatz auf Wasser gegossen, mit verdünnter Salzsäure angesäuert und der anfallende Niederschlag abgesaugt. Man erhielt 0,96 g (42 %) Produkt, Schmp. > 225°C.

### Beispiel 36

N-(1-(1-Carboxymethyl-5,6,7,8-tetrahydrobenzo[f]chinoxalin-2,3-(1H,4H)-dion-9-yl)-pyrrol-3-yl)methyl-N'-(4-nitrophenyl)-guanidin

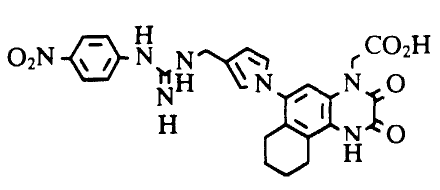

1,8 g (4,8 mMol) des Produktes 27 und 1,6 g (4,8 mMol) S-Methyl-N-(4-nitrophenyl)-isothioharnstoff-hydrojodid wurden analog Vorschrift 35 umgesetzt. Ausbeute: 1,4 g (54 %), Schmp. > 240°C.

¹H-NMR (D₆-DMSO): δ = 1,5 (2H); 1,7 (2h); 2,3 (2H); 2,7 (2H); 4,3 (2H); 4,7 (2H); 6,2 (1H); 6,7 (1H); 6,8 - 7,0 (2H); 7,3 (2H); 8,2 (2H); 8,4 (breit); 9,5 (breit) und ca. 11,2 (breit) ppm.

### Beispiel 37

N-(1-(1-Hydroxy-5,6,7,8-tetrahydrobenzo[f]chinoxalin-2,3-(1H,4H)-dion-9-yl)-pyrrol-3-yl)methyl-N'-(4-trifluormethylphenyl)-harnstoff

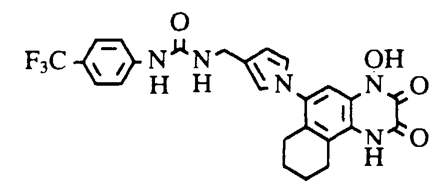

1,5 g (4,5 mMol) des Produktes 5 und 0,9 g (4,7 mMol) 4-Trifluormethylphenylisocyanat wurden analog Beispiel 6 umgesetzt. Ausbeute: 2,1 g (91%), Schmp. : > 215°C.

¹H-NMR (D₆-DMSO): δ =1,5 (2H); 1,7 (2H); 2,4 (2H); 2,7 (2H); 4,1 (2H); 6,1 (1H); 6,6 (1H); 6,8 (2H); 7,1 (1H); 7,5 - 7,7 (4H); 9,0 (1H) und ca. 11,3 (breit) ppm.

### Beispiel 38

N-(1-(1-Carboxymethyl-5,6,7,8-tetrahydrobenzo[f]chinoxalin-2,3-(1H, 4H)-dion-9-yl)-pyrrol-3-yl)methyl-N'- (4-trifluormethylphenyl)harnstoff

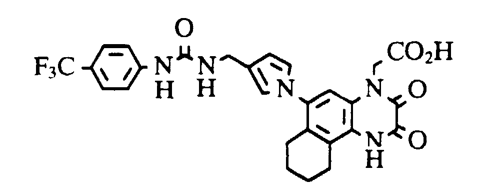

1,3 g (3,5 mMol) des Produktes 27 und 0,68 g (3,7 mMol) 4-Trifluormethylphenylisocyanat wurden analog Beispiel 6 umgesetzt. Ausbeute: 1,4 g (72 %), Schmp. > 210°C.

¹H-NMR (D₆-DMSO): δ = 1,5 (2H);1,7 (2H); 2,4 (2H); 2,7 (2H); 4,1 (2H); 4,7 (2H); 6,1 (1H); 6,7 (3H); 6,9 (1H); 7,4 - 7,6 (4H); 9,1 (1H); 10,7 (1H) und 11,2 (breit) ppm.

### Beispiel 39

1-Ethoxycarbonylmethyl-9-(3-(4-(4-nitrophenyl)-piperazin-1-yl)pyrrol-1-yl)-5,6,7,8-tetrahydrobenzo[f]chinoxalin-2,3-(1H, 4H) dion

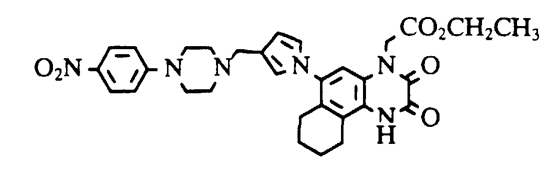

1,24 g (3,1 mMol) des Produktes 20 und 1,3 g (6,3 mMol) 4-Nitrophenyl-piperazin wurden analog Beispiel 14 umgesetzt. Ausbeute: 1,1 g (59 %), Schmp. 229°C.

¹H-NMR (D₆-DMSO): δ = 1,2 (3H); 1,7 2H); 1,8 (2H); 2,4 (2H); 2,9 (2H); 3,3 - 3,7 (8H); 4,2 (2H); 5,0 (2H); 6,2 (1H); 6,8 (2H), 7,0 (2H); 7,1 (1H), 8,0 (2H) und ca. 11,5 (1H) ppm.

### Beispiel 40

9-(3-Carboxy-1-pyrrolyl)-1-ethoxycarbonylmethyl-5,6,7,8-tetrahydrobenzo[f]chinoxalin-2,3-(1H,4H)-dion

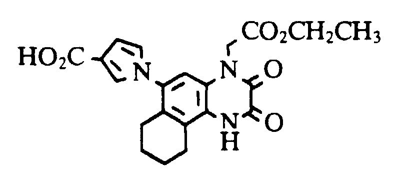

0,52 g (1,3 mMol) des Produktes 20 und 0,49 g (1,3 mMol) Dicyclohexano-18-crown-6 wurden in 20 ml Aceton gelöst und unter Rückfluß erhitzt. Danach wurden 0,83 g (5,3 mMol) Kaliumpermanganat portionsweise zugegeben und noch weitere 30 min gekocht. Man gab 10 ml Wasser hinzu und kochte für weitere 15 min. Danach wurde filtriert und der Niederschlag mit verdünnter Salzsäure und Essigester gewaschen. Die wäßrigen Phasen wurden mit Wasser verdünnt und mit Essigester extrahiert. Die vereinigten Essigesterphasen wurden anschließend mit wäßriger Natriumhydrogenkarbonat-Lösung extrahiert. Diese wurde mit Salzsäure angesäuert und erneut mit Essigester extrahiert. Danach wurde diese organische Phase getrocknet und im Vakuum eingeengt. Ausbeute: 0,2 g (35 %), Schmp. : 282°C.

¹H-NMR (D₆-DMSO): δ = 1,2 (3H); 1,7 (2H); 1,8 (2H); 2,4 (2H); 2,8 (2H); 4,2 (2H); 5,0 (2H); 6,6 (1H), 6,9 (1H); 7,3 (1H); 7,4 (1H); 11,3 (1H) und ca. 13 (1H) ppm.

### Beispiel 41

1-Ethoxycarbonylmethoxy-9-(3-trifluoracetamidomethyl-1-pyrrolyl)-5,6,7,8-tetrahydrobenzo[f]chinoxalin-2,3-(1H,4H)-dion

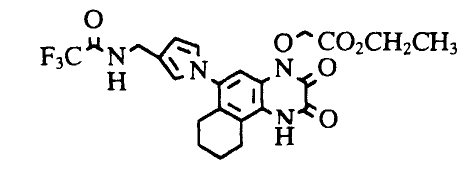

2,5 g (5,9 mMol) des Produktes 4 und 1,5 g (8,9 mMol) Bromessigsäureethylester wurden analog Beispiel 8 umgesetzt. Ausbeute: 2,8g (92 %), Schmp. 199°C.

¹H-NMR (D₆-DMSO): δ = 1,2 (3H), 1,7 (2H); 1,8 (2H); 2,4 (2H); 2,8 (2H), 4,2 (2H); 4,3 (2H); 5,0 (2H); 6,2 (1H); 6,9 (2H); 7,3 (1H); 9,8 (1H) und 11,3 (1H) ppm.

### Beispiel 42

1-Ethoxycarbonylmethyl-9-(3-(4-nitrobenzyl-carbamoyl)-1-pyrrolyl)-5,6,7,8-tetrahydrobenzo[f]chinoxalin-2,3-(1H,4H)-dion

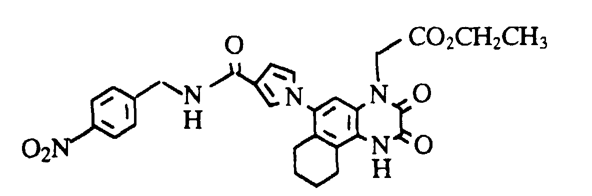

1,5 g (3,6 mMol) Substanz des Beispiels 40 wurden in 50 ml wasserfreiem Dimethylformamid gelöst und bei Raumtemperatur mit 0,73 g (4,5 mMol) Carbonyldiimidazol versetzt. Man rührte 30 min bei Raumtemperatur und weitere 30 min bei 50°C. Anschließend wurden 1,1 g (7,3 mMol) 4-Nitrobenzylamin zugegeben und 1 h bei 80°C gerührt. Danach wurde das Lösungsmittel im Vakuum entfernt und der Rückstand mit verdünnter Salzsäure behandelt. Der anfallende Festkörper wurde abgesaugt. Man erhielt 1,5 g (74 %) Produkt, Schmp. 165°C.

¹H-NMR (D₆-DMSO): δ =1,2 (3H), 1,6 (2H); 1,8 (2H); 2,4 (2H); 2,8 (2H), 4,1 (2H); 4,5 (2H); 5,0 (2H); 6,7 (1H); 6,9 (2H); 7,2 (1H); 7,4-7,7 (3H), 8,1-8,3 (2H); 8,6 (1H) und 10,8 (1H) ppm.

### Beispiel 43

9-(3 -Aminomethyl-1-pyrrolyl)-1-carboxymethyloxy-5,6,7,8-tetrahydrobenzo[f]chinoxalin-2,3(1H,4H)-dion

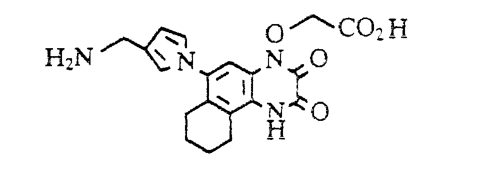

2,1 g (4,2 mMol) Substanz des Beispiel 41 wurden mit 0,5 g (21 mMol) Lithiumhydroxid analog Beispiel 10 umgesetzt. Man erhielt 1,4 g (85 %) Produkt, Schmp. >300°C.

¹H-NMR (D₆-DMSO): δ = 1,6 (2H), 1,75 (2H); 2,4 (2H); 2,7 (2H), 3,8 (2H); 4,3 (2H); 6,3 (1H); 6,8 (1H); 7,0 (1H); 7,5 (1H); 8,5 (breit) und 10,7 (1H) ppm.

### Beispiel 44

1-Carboxymethyl-9-(3-(4-(4-nitrophenyl)piperazin-1-yl)methyl-pyrrol-1-yl)-5,6,7,8-tetrahydrobenzo[f]chinoxalin-2,3-(1H,4H)-dion

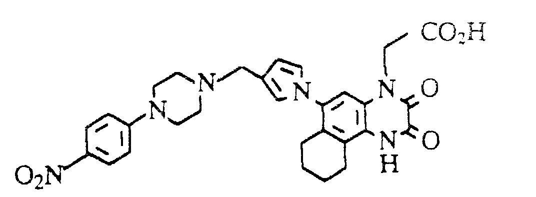

1 g (1,7 mMol) Substanz des Beispiels 39 wurden mit 0,12 g (5,1 mMol) Lithiumhydroxid analog Beispiel 10 umgesetzt. Man erhielt 0,9 g (95 %) Produkt, Schmp. >300°C.

¹H-NMR (D₆-DMSO): δ = 1,6 (2H),1,8 (2H); 2,4 (2H); 2,8 (2H), 3,0-4,0 (8H); 4,1 (2H); 4,9 (2H); 6,4 (1H); 6,9 (1H); 7,0-7,2 (3H); 8,1 (2H) und 10,8 (1H) ppm.

### Beispiel 45

1-Carboxymethyl-9-(4-(4-nitrobenzylcarbamoyl)-pyrrol-1-yl)-5,6,7,8-tetrahydrobenzo[f]chinoxalin-2,3-(1H,4H)-dion

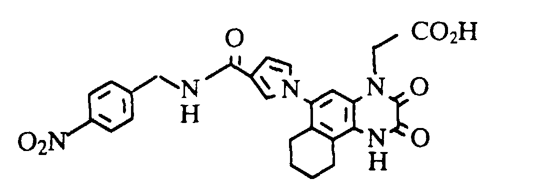

0,9 g (1,6 mMol) Substanz des Beispiels 42 wurden mit 0,12 g (4,9 mMol) Lithiumhydroxid analog Beispiel 10 umgesetzt. Man erhielt 0,7 g (81 %) Produkt, Schmp. >220°C (Zersetzung).

¹H-NMR (D₆-DMSO): δ = 1,6 (2H); 1,8 (2H); 2,4 (2H); 2,8 (2H), 4,6 (2H); 4,9 (2H); 6,7 (1H); 6,9 (2H); 7,2 (1H); 7,4 (1H), 7,6 (2H); 8,2 (2H); 8,6 (1H), 11,3 (1H) und ca. 13 (breit) ppm.

### Beispiel 46

1-Carboxymethyl-9-(3-carboxypyrrol-1-yl)5,6,7,8-tetrahydrobenzo[f]chinoxalin-2,3-(1H,4H)-dion

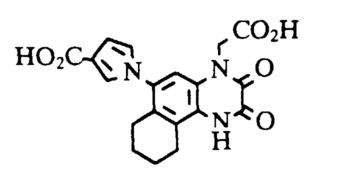

1,8 g (4,3 mMol) Substanz des Beispiels 40 und 0,41 g (17,2 mMol) Lithiumhydroxid wurden analog Beispiel 10 umgesetzt. Man erhielt 11,3 g (80 %) Produkt, Schmp. >245°C (Zersetzung).

¹H-NMR (D₆-DMSO): δ = 1,6 (2H); 1,8 (2H); 2,4 (2H); 2,8 (2H), 4,9 (2H); 6,6 (1H); 6,9 (1H); 7,2 (1H); 7,4 (1H), 11,3 (1H) und ca. 12,5 (breit) ppm.

### Beispiel 47

N(1-(1-Carboxymethyl-5,6,7,8-tetrahydrobenzo[f]chinoxalin-2,3-(1H,4H)-dion-9-yl)-pyrrol-3-ylmethyl)-N'-(3-ethoxycarbonylphenyl)-harnstoff

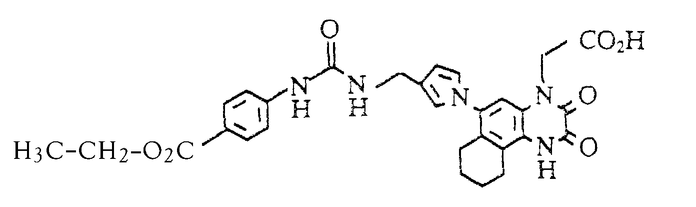

2,0 g (5 mMol) Substanz des Beispiels 27 und 1,1 g (5,5 mMol) 3-Ethoxycarbonylphenylisocyanat wurden analog beispiel 29 umgesetzt. Man erhielt 1,4 g (50 %) Produkt, Schmp. >200°C (Zersetzung).

¹H-NMR (D₆-DMSO): δ =1,3 (3H); 1,6 (2H); 1,8 (2H); 2,45 (2H), 2,8 (2H); 4,2 (2H); 4,3 (2H); 4,9 (2H); 6,2 (1H), 6,3 (1H), 6,8 (2H); 7,0 (2H); 7,4 (1H); 7,5 (1H); 7,6 (1H); 8,1 (1H); 8,7 (1H); 11,3 (1H) und ca. 13,2 (breit) ppm.

### Beispiel 48

1-Ethoxycarbonylmethyl-9-(2-trifluoroacetamidomethylpyrrol-1-yl)-5,6,7,8-benzo[f]chinoxalin-2,3-(1H,4H)-dion 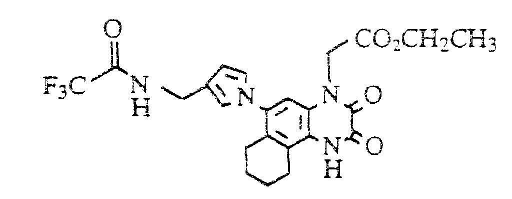
a) (N-((2,5-Dimethoxy-tetrahydrofuran-2-yl)methyl)-trifluoressigsäureamid
   25,0 g (155 mMol) 2-Aminomethyl-2,5-dimethoxy-tetrahydrofuran, 15,7 g (155 mMol) Triethylamin und 1 Spatelspitze 4-(N,N-Dimethylamino)pyridin wurden in 200 ml Ether gelöst. Bei 0 bis 5°C wurden 32,6 g (155 mMol) Trifluoressigsäureanhydrid zugetropft. Man ließ alles 1 h rühren. Danach wurde die Etherphase mit Wasser gewaschen, getrocknet und im Vakuum eingeengt. Man erhielt 32 g (80 %) eines Rohproduktes, das so weiter eingesetzt wurde.
b) 1-Ethoxycarbonylmethyl-9-(2-trifluoracetamidomethylpyrrol-1-yl)-5,6,7,8-benzo[f]chinoxalin-2,3-(1H,4H)-dion
   1,5 g (4,7 mMol) Substanz des Beispiels und 1,5 g (5,9 mMol) des gemäß a) erhaltenen Produktes wurden analog Beispiel 1d umgesetzt. Man erhielt 1,8 g (75 %) Produkt, Schmp. >120°C (Zersetzung).

¹H-NMR (D₆-DMSO): δ =1,2 (3H);1,5 (1H); 1,65 (1H); 1,7 (1H), 1,8 (1H); 2,1 (1H); 2,3 (1H); 2,7-2,9 (2H); 4,0 (1H), 4,1 (3H); 5,0 (2H); 6,2 (2H); 6,7 (1H), 7,3 (1H); 9,5 (1H) und 11,3 (1H) ppm.

### Beispiel 49

N-(1-(1-Carboxymethyloxy-5,6,7,8-tetrahydrobenzo[f]chinoxalin-2,3-(1H,4H) -dion-9-yl)-pyrrol-3-ylmethyl)-N'-(4-nitrophenyl)-harnstoff

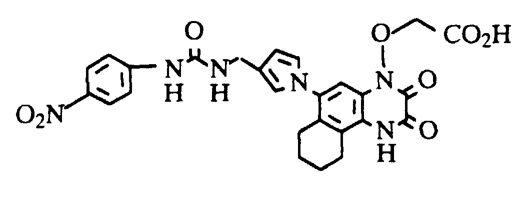

1,0 g (2,5 mMol) Substanz des Beispiels 43 und 0,45 g (2,75 mMol) 4-Nitrophenylisocyanat wurden analog Beispiel 29 umgesetzt. Man erhielt 0,4 g (32 %) Produkt, Schmp. >215°C (Zersetzung).

¹H-NMR (D₆-DMSO): δ =1,7 (2H); 1,9 (2H); 2,6 (2H); 2,9 (2H), 4,4 (2H); 5,0 (2H); 6,3 (1H); 6,8 (1H); 7,6 (3H), 7,7 (1H); 8,1 (2H) und 8,2 (1H) ppm.

### Beispiel 50

9-(3-Benzylcarbamoyl-pyrrol-1-yl)-1-ethoxycarbonylmethyl-5,6,7,8-tetrahydrobenzo[f]chinoxalin-2,3-(1H,4H)-dion

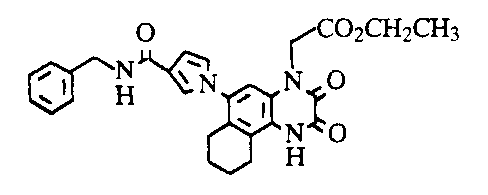

2,0 g (4,9 mMol) Substanz des Beispiels 40 und 1,0 g (9,7 mMol) Benzylamin wurden analog Beispiel 42 umgesetzt. Man erhielt 1,5 g (59 %) Produkt, Schmp. >100°C (Zersetzung).

¹H-NMR (D₆-DMSO) : δ = 1,2 (3H) ; 1,6 (2H) ; 1,8 (2H); 2,4 (2H), 2,8 (2H); 4,1 (2H); 4,4 (2H); 5,0 (2H); 6,7 (1H), 6,9 (1H); 7,1-7,5 (6H), 8,4 (1H) und ca. 11 (breit) ppm.

### Beispiel 51

1-Benzyloxy-9-(3-formyl-pyrrol-1-yl)-5,6,7,8-tetrahydrobenzo[f]chinoxalin-2,3-dion

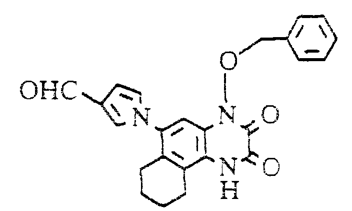

6,6 g (20 mMol) Substanz des Beispiel 1 wurden Beispiel 8 mit 5,2 g (30,6 mMol) Benzylbromid umgesetzt. Man erhielt 8 g (94 %) Produkt, Schmp. 230°C (Zersetzung).

¹H-NMR (D₆-DMSO): δ = 1,6 (2H); 1,8 (2H); 2,4 (2H), 2,8 (2H); 5,2 (2H); 6,6 (1H); 7,0 (1H); 7,1 (1H), 7,4 (1H); 7,6 (2H), 7,7 (1H), 9,8 (1H) und 11,4 (breit) ppm.

### Beispiel 52

N-(1-(1-Ethoxycarbonylmethyl-5,6,7,8-tetrahydrobenzo[f]chinoxalin-2,3-dion-9-yl)pyrrol-3-yl-methyl)-N'-(4-ethoxycarbonylphenyl)-harnstoff 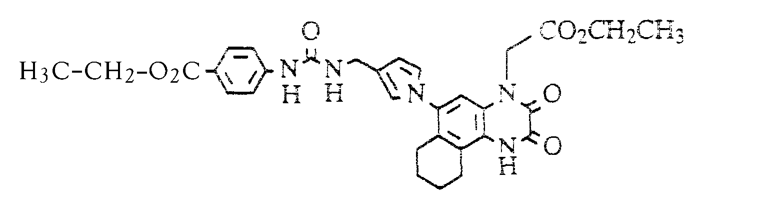
a) N'-(2,5-Dimethoxy-tetrahydrofuran-3-yl)methyl-N-(4-ethoxycarbonyl)-harnstoff
   25 g (0,16 Mol) 3-Aminomethyl-2,5-dimethoxy-tetrahydrofuran (DE 26 45 234; CA 89, 24130) wurden in 200 ml wasserfreiem Tetrahydrofuran (DE 2,645,234; CA, 89, 24130) wurden in 200 ml wasserfreiem Tetrahydorfuran gelöst und bei 0°C mit 30 g (0,15 Mol) 4-Ethoxycarbonylphenylisocyanat, das in 100 ml wasserfreiem Tetrahydorfuran gelöst war, tropfenweise versetzt. Nach 30 min wurde das Reaktionsgemisch im Vakuum eingeengt und ungereinigt weiter verwendet. Man erhielt 57 g.
b) N-(1-(1-Ethoxycarbonylmethyl-5,6,7,8-tetrahydrobenzo[f]chinoxalin-2,3-dion-9-yl)-pyrrol-3-yl)methyl-N'-(4-ethoxycarbonylphenyl)-harnstoff
   1,5 g (3,9 mMol) Substanz des Beispiels 26 g und 1,2 g (4,3 mMol) der gemäß a) erhaltenen Substanz wurden analog 26 h umgesetzt. Man erhielt 1,1 g (40 %) Produkt, Schmp. >160°C (Zersetzung).
   ¹H-NMR (D₆-DMSO): δ =1,1-1,4 (6H); 1,6-1,8 (4H); 2,4 (2H); 2,8 (2H), 4,1-4,4 (6H); 5,0 (2H); 6,2 (1H); 6,5 (1H); 6,8 (2H), 7,1 (1H); 7,5 (2H), 7,6 (1H); 7,8 (2H), 8,9 (1H) und 11,3 (1H) ppm.

### Beispiel 53

N-(1-(1-Carboxymethyl-5,6,7,8-tetrahydrobenzo[f]chinoxalin-2,3-dion-9-yl)pyrrol-3-yl)-methyl-N'-(3-carboxyphenyl)-harnstoff

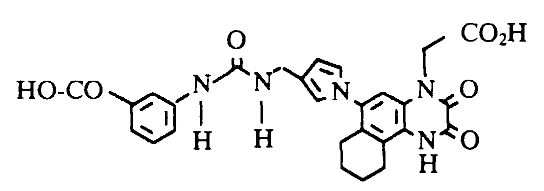

0,9 g (1,6 mMol) Substanz des Beispiels 47 und 0,15 g (6,2 mMol) Lithiumhydroxid wurden analog Beispiel 10 zugesetzt. Man erhielt 0,7 g (87 %) Produkt, Schmp. >210°C (Zersetzung).

¹H-NMR (D₆-DMSO): δ = 1,6 (3H); 1,8 (2H); 2,4 (2H), 2,8 (2H); 4,2 (2H); 4,9 (2H); 6,2 (2H); 6,4 (1H), 6,8 (1H); 7,0 (1H), 7,3 (1H), 7,5 (1H); 7,6 (1H); 8,1 (1H), 8,7 (1H) und 11,3 (breit) ppm.

### Beispiel 54

9-(2-Aminomethyl-pyrrol-1-yl)-1-carboxymethyl-5,6,7,8-tetrahydrobenzo[f]chinoxalin-2,3-(1H,4H)-dion

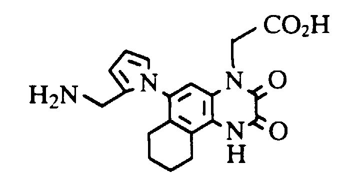

1,2 g (2,5 mMol) des Beispiels 48 und 0,24 g (10 mMol) Lithiumhydroxid wurden analog Beispiel 10 umgesetzt. Man erhielt 0,7 g (76 %) Produkt, Schmp. >285°C (Zersetzung).

¹H-NMR (D₆-DMSO): δ = 1,5-1,9 (4H); 2,0 (1H); 2,3 (1H), 2,8 (2H); 3,4+3.8 (2H); 4,5+4,8 (1H); 6,2 (1H); 6,4 (1H), 6,8 (1H) und 7,1 (1H) ppm.

### Beispiel 55

9-(3-Benzylcarbonyl-pyrrol-1-yl)-1-carboxymethyl-5,6,7,8-tetrahydrobenzo[f]chinoxalin-2,3-(1H,4H)-dion

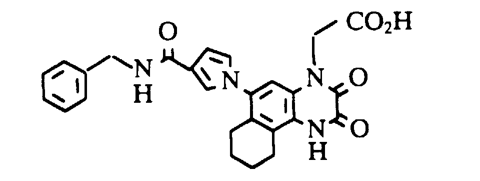

0,9 g (1,7 mMol) Substanz des Beispiels 50 und 0,12 g (5,2 mMol) Lithiumhydroxid wurden analog Beispiel 10 umgesetzt. Man erhielt 0,7 g (81 %) Produkt, Schmp. >200°C (Zersetzung).

¹H-NMR (D₆-DMSO) : = δ 1,6 (2H); 1,8 (2H, 2,4 (2H), 2,8 (2H); 4,4 (2H); 4,9 (2H); 6,7 (1H); 6,9 (1H), 7,1-7,5 (6H); 8,4 (1H) und 7,1 (1H) ppm.

### Beispiel 56

1-Benzyloxy-9(3-carboxy-pyrrol-1-yl)-5,6,7,8-tetrahydrobenzo[f]chinoxalin-2,3-(1H,4H)-dion

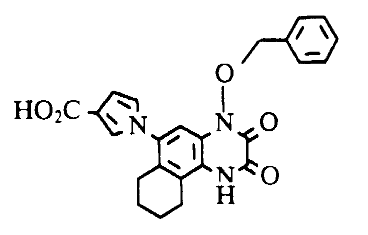

7,2 g (17 mMol) des Beispiels 51 wurden analog Beispiel 40 mit 10,8 g (68 mMol) Kaliumpermanganat oxidiert. Man erhielt 3,0 g (40 %) Produkt, Schmp. >170°C (Zersetzung).

¹H-NMR (D₆-DMSO): δ = 1,6 (2H); 1,8 (2H); 2,4 (2H), 2,8 (2H); 5,2 (2H); 6,6 (1H); 6,9 (1H); 7,0 (1H), 7,4-7,6 (6H); 11,4 (1H) und ca. 12 (breit) ppm.

### Beispiel 57

9-(3-Carboxy-pyrrol-1-yl)-1-hydroxy-5,6,7,8-tetrahydrobenzo[f]chinoxalin-2,3-(1H,4H)-dion

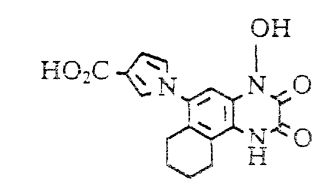

2,6 (6,0 mMol) Substanz des Beispiels 56 wurden in 100 ml Dimethylformamid gelöst und nach Zugabe von 0,5 g Palladium/Kohle (10 %ig) mit Wasserstoff hydriert. Anschließend wurde filtriert und das Filtrat im Vakuum eingeengt. Der Rückstand wurde mit Wasser behandelt und der Festkörper abgesaugt. Man erhielt 2,1 g (100 %) des Produktes, Schmp. >300°C.

¹H-NMR (D₆-DMSO): δ = 1,6 (2H); 1,8 (2H); 2,4 (2H), 2,8 (2H); 6,6 (1H); 6,9 (1H); 7,2 (1H), 7,5 (1H); 11,4 (1H), 11,8 (breit) und 1,02 (breit) ppm.

### Beispiel 58

N'-(4-Ethoxycarbonylphenyl)-N-(1-(1-hydroxy-5,6,7,8-tetrahydrobenzo[f]chinoxalin-2,3-(1h; 4H)-dion-9-yl)-pyrrol-3-yl)methyl-harnstoff

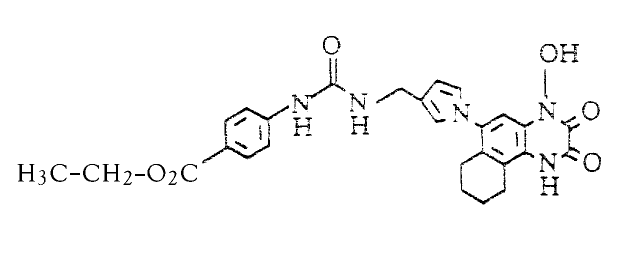

2,0 g (6 mMol) Substanz des Beispiels 5 wurden analog Beispiel 6 mit 1,2 g (6 mMol) 4-Ethoxycarbonylphenylisocyanat umgesetzt. Man erhielt 2,7 g (87 %) Produkt, Schmp. >220°C (Zersetzung).

¹H-NMR (D₆-DMSO): δ = 1,3 (3H); 1,6 (2H); 1,8 (2H), 2,4 (2H); 2,8 (2H); 4,2 (2H); 4,3 (2H), 6,2 (1H); 6,6 (1H), 6,8 (2H); 7,2 (1H); 7,5 (2H); 7,8 (2H) und 9,0 (1H) ppm.

### Beispiel 59

N'-(4-Carboxyphenyl)-N-(1-(1-hydroxy-5,6,7,8-tetrahydrobenzo[f]chinoxalin-2,3-(1H,4H) -dion-9-yl)-pyrrol-3-yl)methylharnstoff

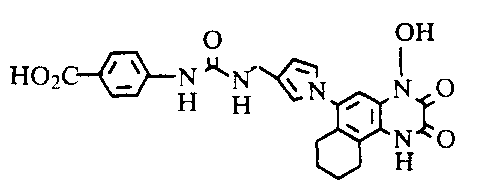

1,9 g (3,7 mMol) Substanz des Beispiels 58 wurden analog Beispiel 10 mit 0,44 g (18,4 mMol) Lithiumhydroxid umgesetzt. Man erhielt 1,4 g (80 %) Produkt, Schmp. >300°C.

¹H-NMR (D₆-DMSO): δ = 1,6 (2H); 1,8 (2H), 2,45 (2H); 2,8 (2H); 4,2 (2H); 6,2 (1H); 6,5 (1H), 6,8 (2H); 7,1 (1H); 7,5 (2H); 7,8 (2H); 8,8 (1H); 11,3 (1H); 11,8 (breit) und 12,5 (breit) ppm.

### Beispiel 60

N-(1-(1-Carboxymethyl-5,6,7,8-tetrahydrobenzo[f]chinoxalin-2,3-(1H,4H)-dion-9-yl)-pyrrol-3-yl)methyl-N'-(4-carboxyphenyl)-harnstoff

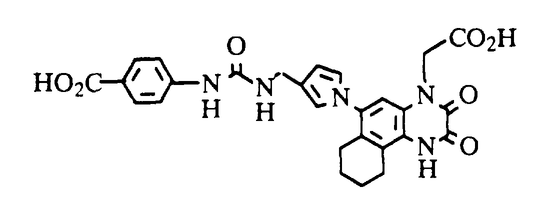

0,7 g (1,3 mMol) Substanz des Beispiels 52 wurden analog Beispiel 10 mit 0,15 g (6,3 mMol) Lithiumhydroxid umgesetzt. Man erhielt 0,55 g (83 %) Produkt, Schmp. >125°C (Zersetzung).

¹H-NMR (D₆-DMSO): δ = 1,3 (2H); 1,5-1,8 (3H); 2,3-2,9 (4H), 4,1-4,3 (2H); 4,7 (2H); 6,2 (1H); 6,7 (2H), 6,9 (1H); 7,4-7,9 (5H), 9,1 (1H), 11,1 (1H) und 11,3 (breit) ppm.

### Beispiel 61

1-Ethoxycarbonylmethyl-9-(3-(4-nitrophenylsulfonamidomethyl)-pyrrol-1-yl)-5,6,7,8-tetrahydrobenzo[f]chinoxalin-2,3-(1H,4H)-dion 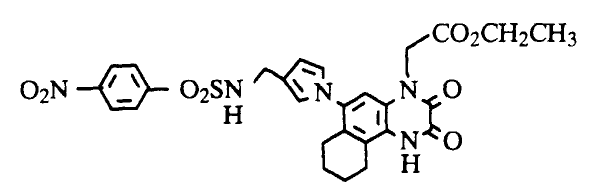
a) N-(2,5-Dimethoxy-tetrahydrofuran-3-yl)methyl-4-nitrophenylsulfonamid
   16,2 g (0,1 Mol) 3-Aminomethyl-2,5-dimethoxy-tetrahydrofuran und 28 ml (0,2 Mol) Triethylamin wurden in 250 ml wasserfreiem Tetrahydrofuran gelöst. Bei 0°C wurden 22,2 g (0,1 Mol) 4-Nitrobenzolsulfonsäurechlorid, gelöst in 100 ml Tetrahydrofuran, zugetropft. Nach 30 min wurde filtriert und das Filtrat im Vakuum eingeengt. Der Rückstand wurde zwischen Essigester und Wasser verteilt, die organische Phase getrocknet und im Vakuum eingeengt. Der Rückstand wurde ungereinigt weiter umgesetzt.
   Man erhielt 28,6 g eines Öles.
b) 1-Ethoxycarbonylmethyl-9-(3-(4-nitrophenylsulfonamidomethyl)-pyrrol-1-yl)-5,6,7,8-tetrahydrobenzo[f]chinoxalin-2,3-(1H,4H)-dion
   2,0 g (6,3 mMol) Substanz des Beispiels 26 g und 2,3 g des gemäß a) erhaltenen Produktes wurden analog Beispiel ld umgesetzt. Man erhielt 2,7 g (73 %) Produkt, Schmp. 218-220°C.
   ¹H-NMR (D₆-DMSO): δ = 1,2 (3H); 1,6 (2H); 1,8 (2H); 2,3 (2H); 2,8 (2H), 4,0 (2H); 4,1 (2H), 5,0 (2H), 6,1 (1H), 6,6 (2H), 7,0 (1H); 8,0 (2H); 8,4 (3H) und 11,3 (1H) ppm.

### Beispiel 62

9-(3-(4-Ethoxycarbonylbenzylcarbamoyl)-pyrrol-1-yl)-1-hydroxy-5,6,7,8-tetrahydrobenzo[f]chinoxalin-2,3-(1H,4H)-dion

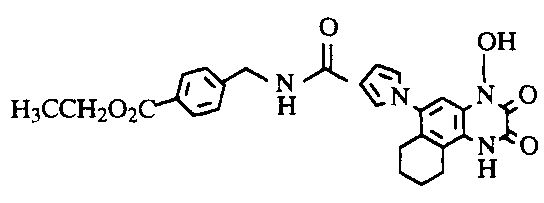

0,75 g (2,2 mMol) Substanz des Beispiels 57 wurden analog Beispiel 42 mit 0,6 g (2,2 mMol) 4-Ethoxycarbonylbenzylammoniumhydrogensulfat umgesetzt. Man erhielt 0,6 g (54 %) Produkt, Schmp. >190°C (Zersetzung).

¹H-NMR (D₆-DMSO): δ = 1,3 (3H); 1,6 (2H); 1,8 (2H); 2,4 (2H); 2,7 (2H), 4,2-4,5 (4H); 6,7 (1H), 6,9 (1H), 6,9 (2H), 7,3-7,5 (3H), 7,9 (2H); 8,5 (1H) und ca. 10,7 (breit) ppm.

### Beispiel 63

1-Carboxymethyl-9-(3-(4-nitrophenylsufonamidomethyl)-pyrrol-1-yl)-5,6,7,8-tetrahydrobenzo[f]chinoxalin-2,3-(1H,4H)-dion

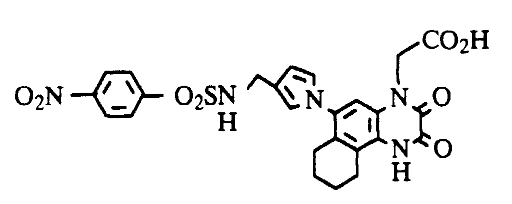

1,8 g (3 mMol) Substanz des Beispiels 61 wurden analog Beispiel 10 mit 0,37 g (16 mMol) Lithiumhydroxid umgesetzt. Man erhielt 1,6 g (93 %) Produkt, Schmp. >150°C (Zersetzung).

¹H-NMR (D₆-DMSO): δ = 1,5 (2H); 1,8 (2H); 2,3 (2H); 2,8 (2H), 4,0 (2H); 4,9 (2H), 6,0 (2H), 6,7 (2H), 6,9 (1H); 8,0-8,6 (6H) und 11,3 (1H) ppm.

## Patentansprüche

1. Pyrrolyl-tetrahydrobenzochinoxalindione der Formel I 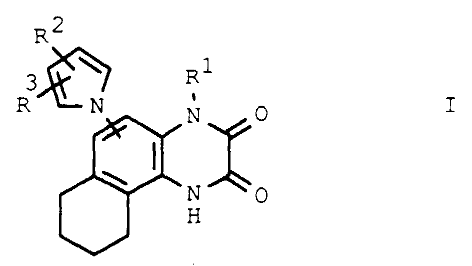 und ihre tautomeren und isomeren Formen, sowie ihre physiologisch verträglichen Salze, worin die Variablen folgende Bedeutung haben:
R¹ Wasserstoff; ein aliphatischer Rest mit 1 oder 2 C-Atomen, der einen oder zwei verschiedene Substituenten der Formel -COOR⁴ tragen kann, worin R⁴ Wasserstoff oder C₁-C₄-Alkyl bedeutet;
-O-R⁶, worin R⁶ Wasserstoff oder eine CH₂-Gruppe ist, die einen der folgenden Reste tragen kann: -COOR⁴ oder Phenyl,
R² Wasserstoff, C₁-C₄-Alkyl oder Phenyl,
R³ Wasserstoff oder der Rest -(CH₂)ₘ-R⁷, wobei m die Zahl 0, 1, 2, 3 oder 4 ist und R⁷ Wasserstoff, C₁-C₄-Alkyl, Phenyl, Phenylsulfonyl, NO₂, CN, -COO-(CH₂)ₙ-R⁸, -CONHSO₂R⁴, -CONH-(CH₂)ₙ-R⁸, -CO-R⁸, -CH=CH-CONHR⁸, -CH=CH-COOR⁸, -CH=NOR⁸, -CH₂-NR⁸R⁹, CH₂NH-CY-(CH₂)ₙR⁹, CH₂NH-CY-X-(CH₂)ₙ-R⁹, CH₂NH-CO-CF₃, CH₂NH-SO₂-R⁹ 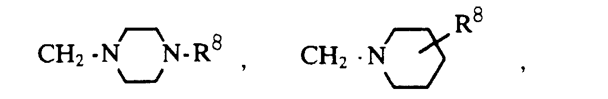 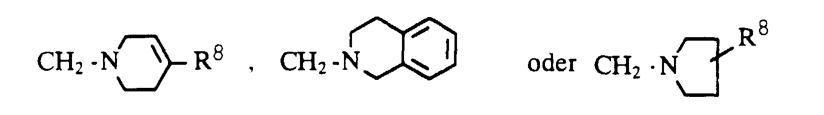 worin X und Y unabhängig voneinander Sauerstoff oder NH sind, n die Zahl 0, 1, 2, 3 oder 4 ist, R⁸ Wasserstoff oder geradliniges und verzweigtes C₁-C₄-Alkyl bedeutet, das durch einen oder zwei Phenyl- oder Pyridyl-Reste substituiert sein kann, und R⁹ Wasserstoff, geradliniges oder verzweigtes C₁-C₆-Alkyl, Phenyl oder Pyridyl bedeutet, wobei alle in R⁸ und R⁹ enthaltenen Phenyl oder Pyridyl-Reste ein oder zwei der folgenden Reste tragen können: O-C₁-C₄-Alkyl, F, Cl, Br, J, C₁-C₄-Alkyl, NO₂, CF₃, -COOR⁵, -CONHR⁵, NH₂, CN, -SO₂Ph, -NHSO₂R⁵, -NHCOR⁵, OH, -SO₂-C₁-C₄-Alkyl, -NHCOCF₃, -SO₂R⁵ und -OCF₃.

2. Pyrrolyl-tetrahydrochinoxalindione der Formel I gemäß Anspruch 1 zur Verwendung bei der Bekämpfung von Krankheiten.

3. Verwendung der Pyrrolyl-tetrahydrobenzochinoxalindione der Formel I gemäß Anspruch 1 zur Herstellung von Arzneimitteln zur Behandlung neurodegenerativer Erkrankungen und neurotoxischer Störungen des zentralen Nervensystems, sowie zur Herstellung von Antiepileptika, Anxiolytika und Antidepressiva.

## Claims

1. A pyrrolyltetrahydrobenzoquinoxalinedione of the formula I 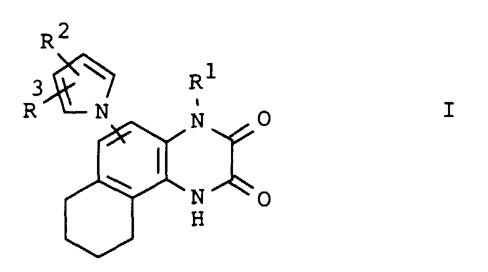 and its tautomeric and isomeric forms, and its physiologically tolerated salts, in which the variables have the following meanings:
R¹ hydrogen; an aliphatic radical which has 1 or 2 carbon atoms and can carry one or two different substituents of the formula -COOR⁴ where R⁴ is hydrogen or C₁-C₄-alkyl;
-O-R⁶ where R⁶ is hydrogen or a CH₂ group which can carry one of the following radicals: -COOR⁴ or phenyl,
R² hydrogen, C₁-C₄-alkyl or phenyl,
R³ hydrogen or the radical -(CH₂)ₘ-R⁷, where m is 0, 1, 2, 3 or 4, and R⁷ is hydrogen, C₁-C₄-alkyl, phenyl, phenylsulfonyl, NO₂, CN, -COO-(CH₂)ₙ-R⁸, -CONHSO₂R⁴, -CONH-(CH₂)ₙ-R⁸, -CO-R⁸, -CH=CH-CONHR⁸, -CH=CH-COOR⁸, -CH=NOR⁸, -CH₂-NR⁸R⁹, CH₂NH-CY-(CH₂)ₙR⁹, CH₂NH-CY-X-(CH₂)ₙ-R⁹, CH₂NH-CO-CF₃, CH₂NH-SO₂-R⁹ 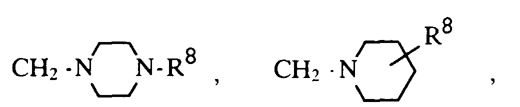 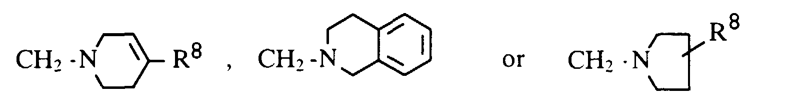 where X and Y are, independently of one another, oxygen or NH, n is 0, 1, 2, 3 or 4, R⁸ is hydrogen or linear and branched C₁-C₄-alkyl which can be substituted by one or two phenyl or pyridyl radicals, and R⁹ is hydrogen, linear or branched C₁-C₆-alkyl, phenyl or pyridyl, where all the phenyl or pyridyl radicals contained in R⁸ and R⁹ can carry one or two of the following radicals: O-C₁-C₄-alkyl, F, Cl, Br, I, C₁-C₄-alkyl, NO₂, CF₃, -COOR⁵, -CONHR⁵, NH₂, CN, -SO₂Ph, -NHSO₂R⁵, -NHCOR⁵, OH, -SO₂-C₁-C₄-alkyl, -NHCOCF₃, -SO₂R⁵ and -OCF₃.

2. A pyrrolyltetrahydrobenzoquinoxalinedione of the formula I as claimed in claim 1 for use for controlling diseases.

3. The use of the pyrrolyltetrahydrobenzoquinoxalinediones of the formula I as claimed in claim 1 for producing drugs for the treatment of neurodegenerative disorders and neurotoxic disturbances of the central nervous system, and for producing antiepileptics, anxiolytics and antidepressants.

## Revendications

1. Pyrrolyl-tétrahydrobenzoquinoxaline-diones de formule I 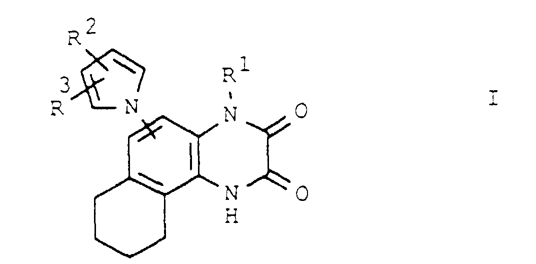 et leurs formes tautomères et isomères ainsi que leurs sels acceptables pour l'usage pharmaceutique, les symboles de la formule ayant les significations suivantes :
R¹ : l'hydrogène ; un groupe aliphatique en C1 ou C2 qui peut porter un ou deux substituants différents de formule -COOR⁴ dans laquelle R⁴ représente l'hydrogène ou un groupe alkyle en C1-C4 ;
un groupe -O-R⁶ dans laquelle R⁶ représente l'hydrogène ou un groupe CH₂ qui peut porter l'un des substituants suivants : -COOR⁴ ou phényle,
R² : représente l'hydrogène, un groupe alkyle en C1-C4 ou phényle,
R³: représente l'hydrogène ou le groupe -(CH₂)ₘ-R⁷ dans lequel m est égal à 0, 1, 2, 3 ou 4 et R⁷ représente l'hydrogène, un groupe alkyle en C1-C4, phényle, phénylsulfonyle, NO₂, CN, - COO-(CH₂)ₙ-R⁸, -CONHSO₂R⁴, -CONH-(CH₂)ₙ-R⁸, -CO-R⁸, -CH=CH-CONHR⁸, -CH=CH-COOR⁸, -CH=NOR⁸, -CH₂-NR⁸R⁹, CH₂NH-CY-(CH₂)ₙR⁹, CH₂NH-CY-X-(CH₂)ₙ-R⁹, CH₂NH-CO-CF₃, CH₂NH-SO₂R⁹ 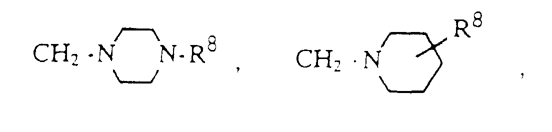 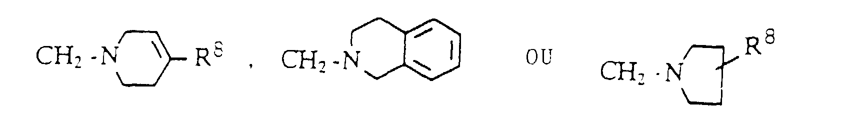 dans lesquels X et Y représentent chacun, indépendamment l'un de l'autre, l'oxygène ou un groupe NH, n est égal à 0, 1, 2, 3 ou 4, R⁸ représente l'hydrogène ou un groupe alkyle à chaîne droite ou ramifiée en C1-C4 qui peut porter un ou deux substituants phényle ou pyridyle, et R⁹ représente l'hydrogène, un groupe alkyle à chaîne droite ou ramifiée en C1-C6, un groupe phényle ou pyridyle, tous les groupes phényle ou pyridyle contenus dans R⁸ et R⁹ pouvant porter un ou deux des substituants suivants : O-alkyle en C1-C4, F, Cl, Br, I, alkyle en C1-C4, NO₂, CF₃, -COOR⁵, -CONHR⁵, NH₂, CN, -SO₂Ph, -NHSO₂R⁵, -NHCOR⁵, OH,-SO₂-alkyle en C1-C4, -NHCOCF₃, -SO₂R⁵ et -OCF₃.

2. Pyrrolyl-tétrahydroquinoxaline-diones de formule I selon la revendication 1 pour l'utilisation dans le traitement de maladies.

3. Utilisation des pyrrolyl-tétrahydrobenzoquinoxalinediones de formule I de la revendication 1 pour la préparation de médicaments prévus pour le traitement des maladies neurodégénératives et des troubles neurotoxiques du système nerveux central, ainsi que pour la préparation de médicaments antiépileptiques, anxiolytiques et antidépressifs.
